# EUROPEAN PATENT APPLICATION

(11) **EP 4 726 393 A1**
(43) Date of publication of application: **15.04.2026**
(21) Application number: 24823379.3
(22) Date of filing: 11.06.2024
(51) Int. Cl.: G01N 33/68, C07K 14/705, C12N 15/44, C12N 15/50, G01N 33/53, G01N 33/569

(54) **IMMUNE REACTION EVALUATION METHOD**

(30) Priority: 12.06.2023 JP 2023096200
(71) Applicant: National University Corporation Kobe University, Kobe-shi, Hyogo 657-8501 (JP)
(72) Inventor: FUNAKOSHI, Yohei, Kobe-shi, Hyogo 657-8501 (JP); YAKUSHIJIN, Kimikazu, Kobe-shi, Hyogo 657-8501 (JP); OHJI, Goh, Kobe-shi, Hyogo 657-8501 (JP); MINAMI, Hironobu, Kobe-shi, Hyogo 657-8501 (JP); MATSUTANI, Takaji, Kyoto-shi, Kyoto 600-8815 (JP)
(74) Representative: Müller-Boré & Partner Patentanwälte PartG mbB
(86) International application number: PCT/JP2024/021195
(87) International publication number: WO 2024/257764

(57) **Abstract**

The purpose of the present invention is to provide a method for selectively identifying an immune reaction to a specific antigen, at the level of antibody sequences, from repertoire data for immune cell receptors. An immune reaction to a specific antigen can be selectively identified, at the level of antibody sequences, by this immune reaction evaluation method, the method including: a step A for preparing repertoire data of sequences for antigen recognition sites in immune cell receptors, wherein the repertoire data is acquired from a sample collected from a subject; and a step B for collating a database of a sequence for an antigen recognition site against a specific antigen with the repertoire data to identify a sequence which is identical to the sequence included in the database or a sequence of which a portion excluding two or less amino acid residues is identical to the sequence included in the database at the level of amino acid sequences.

## Description

### Technical Field

The present invention relates to a method for evaluating an immune reaction.

### Background Art

Since coronavirus infectious disease (COVID-19) caused by severe acute respiratory syndrome coronavirus-2 (SARS-CoV-2) poses a threat to the lives of patients with hematologic malignancies, vaccination with mRNA SARS-CoV-2 is generally recommended by hematologists (NPLs 1 and 2). However, it has been reported that some patients with hematologic malignancies, such as patients subjected to hematopoietic stem cell transplantation (HSCT) and patients subjected to B cell depletion therapy, fail to achieve a sufficient humoral response after vaccination (NPLs 3 to 6). Even after the occurrence of the omicron variant of SARS-CoV-2, which is considered to be less pathogenic (NPL 7), there are reports that the hospitalization and death rates of immunodeficient patients are still high, and particularly high in patients having a poor reaction due to vaccination (NPLs 8 and 9).

For such patients, Food and Drug Administration (FDA) granted emergency use for tixagevimab/cilgavimab in December 2021. This agent contains a neutralizing monoclonal antibody against a different epitope of a receptor-binding domain of the SARS-CoV-2 spike protein and is used as a pre-exposure prophylactic agent. Based on the results of the PROVENT test, tixagevimab/cilgavimab is considered to be effective for preventing exacerbation and shortening the hospital stay for immunodeficient patients (NPLs 10 and 11). However, a research group has reported that patients with hematologic malignancies, particularly patients subjected to B cell depletion therapy or hematopoietic stem cell transplantation are at a risk of breakthrough infection even with tixagevimab/cilgavimab (NPL 12). Current information shows that tixagevimab/cilgavimab has low activity against specific omicron variants such as BQ.1 and XBB (NPLs 13 and 14). The activity of tixagevimab/cilgavimab, which was developed prior to the emergence of the Omicron variants, has reduced against the current predominant strains in certain countries (NPLs 13 and 14).

Therefore, FDA emphasizes that tixagevimab/cilgavimab is not an alternative to vaccination and that patients who have been administered with tixagevimab/cilgavimab should be recommended to receive routine vaccination. Hematologists propose that these patients maintain various aspects of prevention of infection prophylactic behavior, vaccination, antiviral antibody drugs, and the like (NPL 1). A study has reported a favorable outcome of COVID-19 due to omicron variants as well as previous strains in a cohort of highly vaccinated hematopoietic stem cell transplantation patients (NPL 15).

Immunogenicity after SARS-CoV-2 vaccination, particularly, humoral immunity, is generally evaluated by enzyme-linked immunosorbent assay (ELISA) using an anti-SARS-CoV-2 spike IgG antibody (NPL 16). Until now, the reactivity of vaccination has been evaluated by measuring antibody titers after vaccination in patients with various immune states such as healthy individuals and cancer patients (NPLs 17 and 18).

### Citation List

### Non Patent Literatures

NPL 1: Vijenthira A, Gong IY, Fox TA, et al. Outcomes of patients with hematologic malignancies and COVID-19: a systematic review and meta-analysis of 3377 patients. Blood. 2020;136(25):2881-2892.
NPL 2: Xhaard A, Xhaard C, D'Aveni M, et al. Risk factors for a severe form of COVID-19 after allogeneic haematopoietic stem cell transplantation: a Societe Francophone de Greffe de Moelle et de Therapie cellulaire (SFGM-TC) multicentre cohort study. Br J Haematol. 2021;192(5):e121-e124.
NPL 3: Dhakal B, Abedin S, Fenske T, et al. Response to SARS-CoV-2 vaccination in patients after hematopoietic cell transplantation and CAR T-cell therapy. Blood. 2021;138(14):1278-1281.
NPL 4: Watanabe M, Yakushijin K, Funakoshi Y, et al. The Safety and Immunogenicity of the BNT162b2 mRNA COVID-19 Vaccine in Japanese Patients after Allogeneic Stem Cell Transplantation. Vaccines (Basel). 2022;10(2).
NPL 5: Funakoshi Y, Yakushijin K, Ohji G, et al. Limited increase in antibody titers following mRNA SARS-CoV-2 vaccination for more than 3 years after final dose of anti-CD20 antibody. Int J Hematol. 2022;115(1):7-10.
NPL 6: Einarsdottir S, Martner A, Nicklasson M, et al. Reduced immunogenicity of a third COVID-19 vaccination among recipients of allogeneic hematopoietic stem cell transplantation. Haematologica. 2022;107(6):1479-1482.
NPL 7: Malahe SRK, Hoek RAS, Dalm V, et al. Clinical Characteristics and Outcomes of Immunocompromised Patients With Coronavirus Disease 2019 Caused by the Omicron Variant: A Prospective, Observational Study. Clin Infect Dis. 2023;76(3):e172-e178.
NPL 8: Taenaka R, Obara T, Kohno K, Aoki K, Ogawa R. Infections with the SARS-CoV-2 Omicron variant show a similar outcome as infections with the previous variants in patients with hematologic malignancies. Ann Hematol. 2022;101(8):1877-1878.
NPL 9: Bahremand T, Yao JA, Mill C, Piszczek J, Grant JM, Smolina K. COVID-19 hospitalisations in immunocompromised individuals in the Omicron era: a population-based observational study using surveillance data in British Columbia, Canada. Lancet Reg Health Am. 2023;20:100461.
NPL 10: Levin MJ, Ustianowski A, De Wit S, et al. Intramuscular AZD7442 (Tixagevimab-Cilgavimab) for Prevention of Covid-19. N Engl J Med. 2022;386(23):2188-2200.
NPL 11: Jondreville L, D'Aveni M, Labussiere-Wallet H, et al. Pre-exposure prophylaxis with tixagevimab/cilgavimab (AZD7442) prevents severe SARS-CoV-2 infection in recipients of allogeneic hematopoietic stem cell transplantation during the Omicron wave: a multicentric retrospective study of SFGM-TC. J Hematol Oncol. 2022;15(1):169.
NPL 12: Davis JA, Granger K, Roubal K, et al. Efficacy of tixagevimab-cilgavimab in preventing SARS-CoV-2 for patients with B-cell malignancies. Blood. 2023;141(2):200-203.
NPL 13: Imai M, Ito M, Kiso M, et al. Efficacy of Antiviral Agents against Omicron Subvariants BQ.1.1 and XBB. N Engl J Med. 2023;388(1):89-91.
NPL 14: Stuver R, Shah GL, Korde NS, et al. Activity of AZD7442 (tixagevimab-cilgavimab) against Omicron SARS-CoV-2 in patients with hematologic malignancies. Cancer Cell. 2022;40(6):590-591.
NPL 15: Tan JY, Wee LE, Tan YH, et al. Favorable outcomes of COVID-19 in vaccinated hematopoietic stem cell transplant recipients: A single-center experience. Transpl Infect Dis. 2023:e14024.
NPL 16: Jung K, Shin S, Nam M, et al. Performance evaluation of three automated quantitative immunoassays and their correlation with a surrogate virus neutralization test in coronavirus disease 19 patients and pre-pandemic controls. J Clin Lab Anal. 2021;35(9):e23921.
NPL 17: Massarweh A, Eliakim-Raz N, Stemmer A, et al. Evaluation of Seropositivity Following BNT162b2 Messenger RNA Vaccination for SARS-CoV-2 in Patients Undergoing Treatment for Cancer. JAMA Oncol. 2021;7(8):1133-1140.
NPL 18: Funakoshi Y, Yakushijin K, Ohji G, et al. Safety and immunogenicity of the COVID-19 vaccine BNT162b2 in patients undergoing chemotherapy for solid cancer. J Infect Chemother. 2022;28(4):516-520.

### Summary of Invention

### Technical Problem

Importantly, since tixagevimab/cilgavimab is an anti-spike IgG antibody per se, in all patients administered with tixagevimab/cilgavimab, the antibody titer by ELISA method is maintained at a considerably higher level over time. Therefore, the administration of tixagevimab/cilgavimab will mask the expression of anti-spike antibodies after vaccination. That is, although vaccination is recommended, there is a problem in that the response to vaccination of these patients cannot be evaluated. The problem in that only the targeted immune reaction cannot be selectively evaluated in this way is not unique under the situation after the administration of tixagevimab/cilgavimab, and is also common under the situation after the administration of all antibody pharmaceutical agents against SARS-CoV-2, such as casirivimab/imdevimab and sotrovimab, and other antibody pharmaceutical agents that may be clinically approved in the future. This problem occurs not only after administration of the antibody drug but also in a situation where the antibody titer is maintained at a high level due to vaccination or infection immediately before. This problem is a universal problem applied to not only to vaccines against SARS-CoV-2 but also to evaluation of the reaction (e.g., infection, cancer immune reaction, or autoimmune reaction) to any vaccine, and further any antigen. However, any technique currently used cannot selectively evaluate only a targeted immune reaction. Under such circumstances, there is a need for a new analysis method capable of evaluating an immune reaction on the basis of the level of mRNA expression, rather than on the basis of the level of protein expression as in the measurement of antibody titer in the conventional ELISA.

As described above, there is a need for a method capable of evaluating the reaction after vaccination at the mRNA level. Recently, in view of the fact that repertoire analysis for determining a large amount of gene sequences of antigen recognition sites in an immune cell receptor (antigen receptor) at a time has become possible by using a next-generation sequencer, the present inventors have considered that repertoire analysis can be used for the above method. However, since immune cell receptors have enormous diversity, it has been extremely difficult to evaluate an immune reaction to a specific antigen alone. The difficulty is so remarkable that it is even impossible to confirm which of the immune cell receptors has expanded after the immune intervention, and in particular, when the immune intervention generates a relatively moderate immune reaction like a vaccine, it is practically impossible to perform the confirmation described above. The reason why the repertoire analysis cannot be simply applied to evaluate the reaction after vaccination at the mRNA level in this way is that the repertoire analysis is a means that essentially evaluates the degree or change of immune diversity, and naturally, it is not assumed that an immune reaction to a specific antigen is selectively specified at the level of an antibody sequence.

An object of the present invention is to provide a method for selectively specifying an immune reaction to a specific antigen, at the mRNA level, from repertoire data for immune cell receptors.

### Solution to Problem

In the background of the recent COVID-19 pandemic, the present inventors have focused that enormous pieces of information such as sequences of SARS-CoV-2 antibodies are accumulated to form a database, and have arrived at a novel idea of selectively specifying an immune reaction to a specific antigen at the level of an antibody sequence from repertoire data of immune cell receptors by using this database as query information for querying the repertoire data of immune cell receptors. That is, the present inventors have found that an immune reaction to a specific antigen can be selectively specified at the level of an antibody sequence by collating repertoire data of immune cell receptors with a database of sequences of antigen recognition sites for a specific antigen. The present invention has been completed by conducting further studies on the basis of these findings.

That is, the present invention provides inventions of the following aspects.
Item 1. A method for evaluating an immune reaction, the method including:
   step A of preparing repertoire data including a group of sequences of antigen recognition sites in immune cell receptors, the repertoire data being acquired from a specimen of a subject; and
   step B of collating a database including a group of sequences of antigen recognition sites in immune cell receptors for a specific antigen with the repertoire data to detect, from the repertoire data, a sequence which is identical to the sequence included in the database at a level of amino acid sequences or a sequence which is identical to the sequence included in the database at a portion excluding two or less amino acid residues.
   This makes it possible to specify a sequence that has reacted immunologically in the living body of the subject at the collection time of the specimen.
Item 2. The method according to item 1, further including step C of deriving the number and/or frequency of sequences detected in the step B in the repertoire data.
Item 3. The method according to item 2, further including step D of confirming a temporal change in the number and/or frequency.
Item 4. The method according to item 3, wherein it is confirmed in the step D whether a time when an increase is observed as the temporal change corresponds to 11 to 20 days or 3 to 10 days after stimulation with the specific antigen.
Item 5. The method according to any one of items 1 to 4, wherein the subject is a subject exposed to the specific antigen.
Item 6. The method according to item 5, wherein the exposure to the specific antigen is vaccination with a vaccine.
Item 7. The method according to item 6, wherein the vaccine is a nucleic acid vaccine.
Item 8. The method according to item 6, wherein the vaccine is a coronavirus vaccine or an influenza vaccine.
Item 9. The method according to item 7 or 8, further including step E of evaluating the scientific validity of the nucleic acid vaccine by confirming additional information listed in the database for the sequence in which an increase is confirmed as the temporal change in the number and/or frequency, wherein
   the additional information is selected from the group consisting of [1] information on the presence or absence of neutralizing activity against an antigen, [2] information on a species or strain of an antigen, and [3] information on an epitope, and
   the evaluating of the scientific validity is selected from the group consisting of [1] evaluating that the nucleic acid vaccine has clinical effectiveness of producing an antibody having neutralizing activity when it is confirmed that the neutralizing activity exists, [2] evaluating that the nucleic acid vaccine is compatible with a target antigen when it is confirmed that the species or strain is the same as a species or strain of the target antigen in design of the nucleic acid vaccine, and [3] evaluating that the nucleic acid vaccine is compatible with a target epitope when it is confirmed that the epitope is the same as the target epitope in design of the nucleic acid vaccine.
Item 10. The method according to any one of items 7 to 9, further including step E' of evaluating that the nucleic acid vaccine has an ability to produce an antibody having an antigen recognition site completely identical to that of an antibody by adaptive immunity by infection, as the scientific validity of the nucleic acid vaccine, when the sequence in which an increase is confirmed as the temporal change in the number and/or frequency is identical to the sequence included in the database at a level of amino acid sequences.
Item 11. The method according to item 5, wherein the exposure to the specific antigen is infection.
Item 12. The method according to item 11, wherein the specific antigen is a coronavirus or an influenza virus.
Item 13. The method according to any one of items 5 to 12, wherein the subject is a subject administered with an antibody drug, vaccinated with a vaccine, and/or infected, and
   the antibody drug, the vaccine, and a pathogen of the infection allow another antibody different from an antibody produced by exposure to the specific antigen to be retained in a body of the subject.
Item 14. The method according to item 13, wherein the specific antigen is a vaccine, and
   the method further includes step F of determining that the vaccine works when the number and/or frequency increases over time.
Item 15. The method according to any one of items 5 to 14, wherein the specimen is collected at a time of activation of mRNA of an immune cell receptor for the specific antigen.
Item 16. The method according to item 15, wherein the time of activation is 11 to 20 days or 3 to 10 days after the exposure.
Item 17. The method according to any one of items 1 to 3, wherein the subject is a subject subjected to cancer immunotherapy.
Item 18. The method according to item 16, wherein the specimen is collected at a time of activation of mRNA of an immune cell receptor for the specific antigen.
Item 19. The method according to item 17, wherein the time of activation is 11 to 20 days or 3 to 10 days after the cancer immunotherapy.
Item 20. The method according to any one of items 1 to 16, wherein the subject is a subject subjected to an immunosuppressive treatment.
Item 21. The method according to item 20, wherein the immunosuppressive treatment is hematopoietic stem cell transplantation or administration of a B cell depletion therapy.
Item 22. The method according to item 21, wherein the immunosuppressive treatment is hematopoietic stem cell transplantation or a B cell depletion therapy, and the method further includes step G of determining the presence or absence of restoration of immune function on the basis of whether or not the temporal change in the number and/or frequency has increased.
Item 23. The method according to any one of items 1 to 16, wherein the subject is a patient with an autoimmune disease.
Item 24. The method according to item 1 or 2, wherein the repertoire data is acquired from a specimen of a subject vaccinated with a vaccine against a previously-encountered viral strain ST1,
   the specific antigen is a viral strain ST2 for which effectiveness by the vaccine is unknown, and
   the method further includes step H of evaluating that the vaccine is also effective against the viral strain ST2 when the detected sequence is present.
Item 25. The method according to any one of items 1 to 24, wherein the database includes sequences of antigen recognition sites in the immune cell receptors for the specific antigen, the sequences of antigen recognition sites in the immune cell receptors collected from a population consisting of objects to be examined in which an immune reaction by the specific antigen has occurred, and the database is obtained by the following steps:
   a step of acquiring, from each specimen of the objects to be examined, repertoire data in time series at a time Tₑₓ of activation of mRNA of an immune cell receptor for the specific antigen, a time T_{bf} before the time of activation and a time T_{af} after the time of activation, the repertoire data including a group of sequences of antigen recognition sites in the immune cell receptors; and
   a step of selecting a sequence of an antigen recognition site in an immune cell receptor at which proliferation is observed at the time Tₑₓ of activation as information to be collected in the database.
Item 26. The method according to item 25, wherein the time Tₑₓ of activation is 11 to 20 days or 3 to 10 days after stimulation with the specific antigen.
Item 27. The method according to any one of items 1 to 26, wherein the immune cell is a T cell or a B cell.

### Advantageous Effects of Invention

According to the present invention, there is provided a method for selectively specifying an immune reaction to a specific antigen, at the mRNA level, from repertoire data for immune cell receptors.

### Brief Description of Drawings

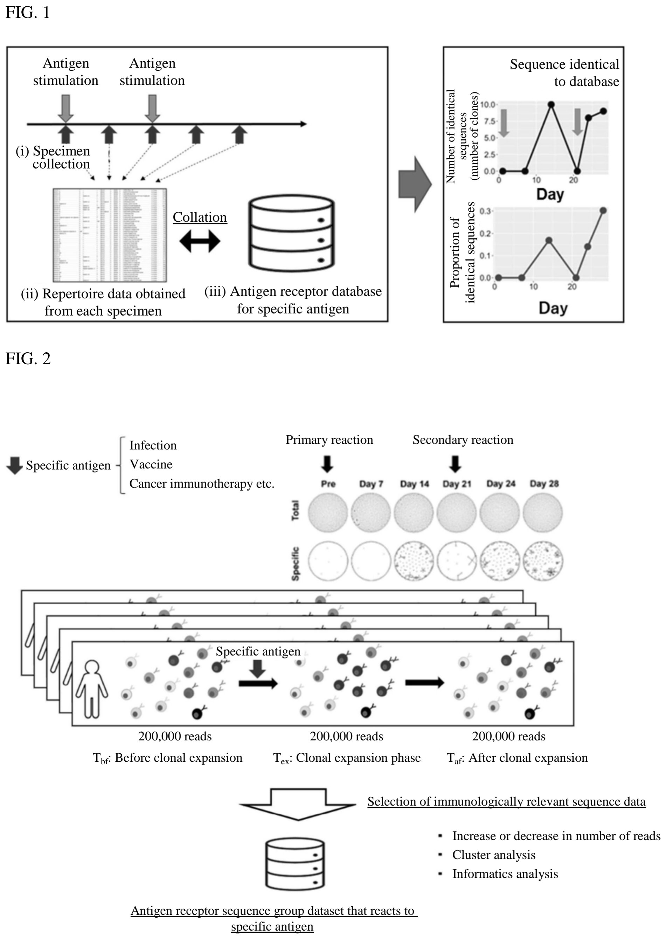

[Fig. 1] Fig. 1 shows the outline of a method for evaluating an immune reaction of the present invention.
[Fig. 2] Fig. 2 shows a conceptual diagram of an example of creating a database applied to the method for evaluating an immune reaction of the present invention.

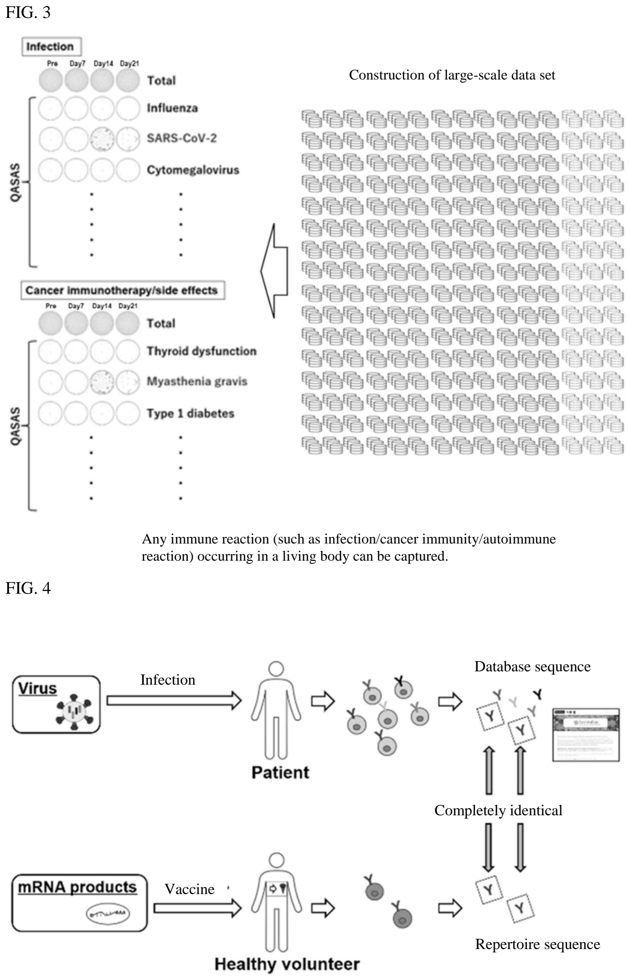

[Fig. 3] Fig. 3 shows a conceptual diagram of an example in which a large-scale data set group is applied as an exhaustive biosensor to the method for evaluating an immune reaction of the present invention.
[Fig. 4] Fig. 4 shows a conceptual diagram of an example of scientific validity of a vaccine (an antibody completely identical to an antibody acquired by a patient by infection with an antigen can be produced in the body of a subject who is a healthy individual by vaccination) that can be evaluated by the method for evaluating an immune reaction of the present invention.

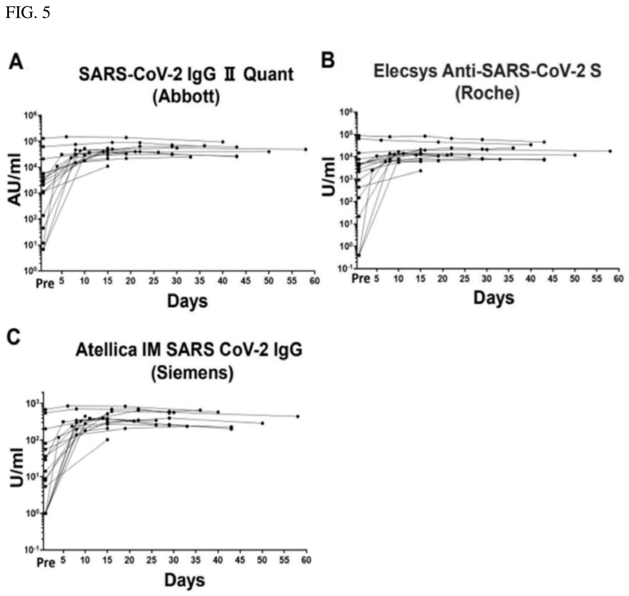

[Fig. 5] Fig. 5 shows changes in anti-spike antibody titer. Anti-spike antibody titers before and after the administration of tixagevimab/cilgavimab were measured in three types of fully automated commercial immunoassays in 17 patients: A) Abbott SARS-CoV-2 IgG II Quant, B) Roche Elecsys anti-SARS-CoV-2 S, and C) Atellica IM SARS CoV-2 IgG.

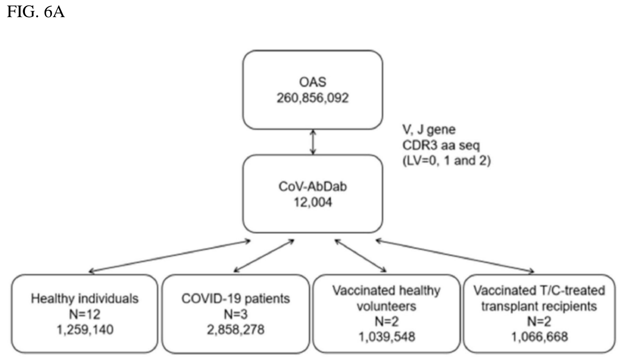

[Fig. 6A] Fig. 6A shows details of study design and samples. The sequence of the CoV-AbDab database was searched for SARS-CoV-2 specific sequences in the OAS public database, healthy individuals (n = 12), COVID-19 patients (n = 3), vaccinated healthy volunteers (n = 2), vaccinated hematopoietic stem cell transplantation patients treated with tixagevimab/cilgavimab (n = 2). The numbers indicate the number of sequences used.

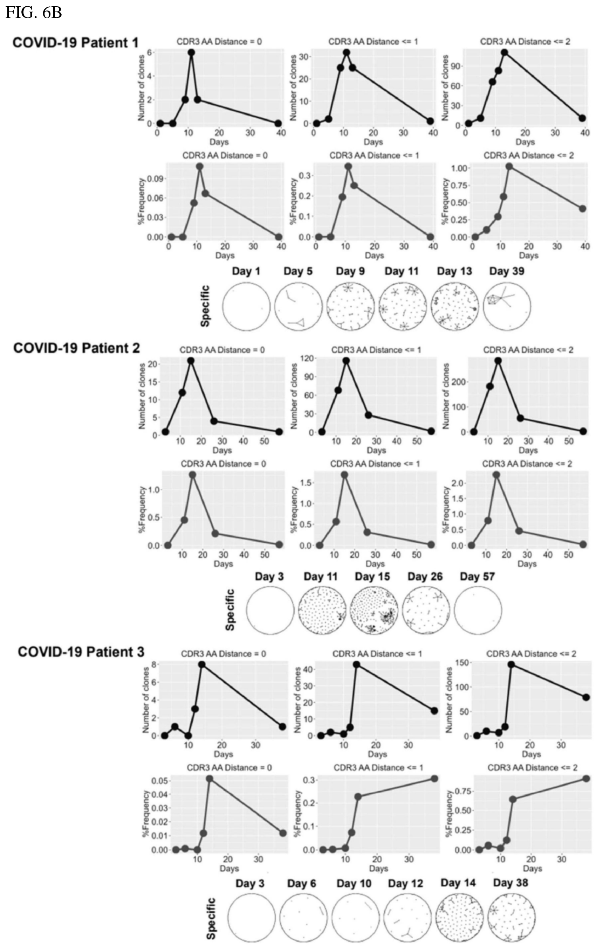

[Fig. 6B] Fig. 6B shows a temporal change in the number and frequency of sequences specific for SARS-CoV-2 in three patients infected with COVID-19. NGS analysis was performed on PBMCs collected over time after SARS-CoV-2 infection. Clone numbers (top) and frequencies (bottom) of SARS-CoV-2 specific sequences with identical V and J genes to the CoV-AbDab sequence and with a Levenshtein distance of the CDR3 amino acid sequence of 0 (left), 1 (middle), or 2 (right) are shown. The plot of the lower circle shows a cluster plot of SARS-CoV-2 specific sequences. A network of up to 1000 SARS-CoV-2 specific sequences by igraph is shown. Each node shows a unique read with identical IGHV, IGHJ, and complementarity determining region 3 (CDR3) amino acid sequences. The nodes were connected by an edge defined by a Levenshtein distance of less than 1 of the CDR3 amino acid sequence. The size of the node was the percent frequency of each unique read.

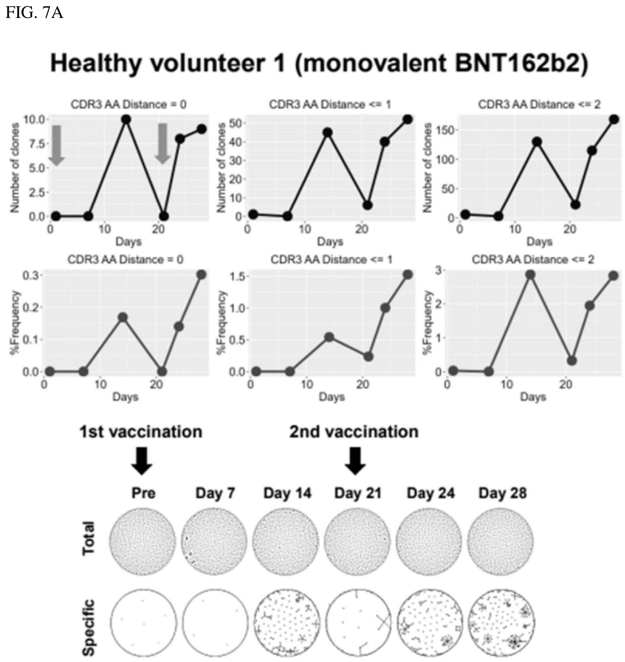

[Fig. 7A] Fig. 7A shows a temporal change in the number, frequency, and cluster of SARS-CoV-2 specific sequences at initial vaccination and after booster vaccination. Healthy volunteer 1 received the first vaccination with an mRNA SARS-CoV-2 vaccine (monovalent BNT162b2), and received the second vaccination 21 days later.

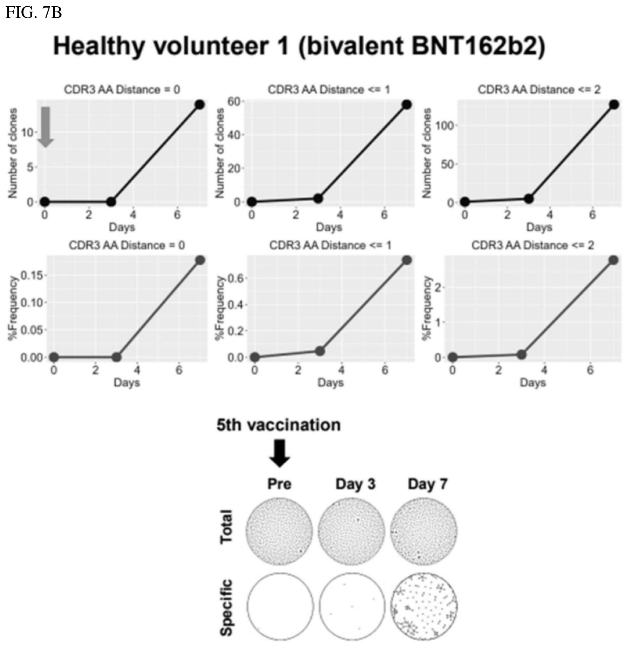

[Fig. 7B] Fig. 7B shows that the fifth vaccination with a bivalent BNT162b2 vaccine was performed after vaccination in Fig. 7A. After these vaccinations, SARS-CoV-2 specific sequences were retrieved from the BCR repertoire data.

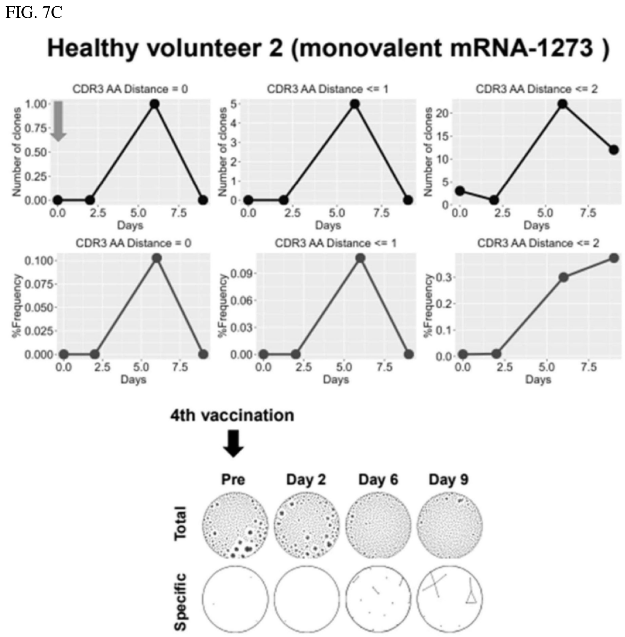

[Fig. 7C] Fig. 7C shows a temporal change in the number, frequency, and cluster of SARS-CoV-2 specific sequences at initial vaccination and after booster vaccination. In Healthy volunteer 2, monovalent mRNA-1273 was administered at the fourth time and then SARS-CoV-2 specific sequences were measured on Days 2, 6, and 9 after booster vaccination.

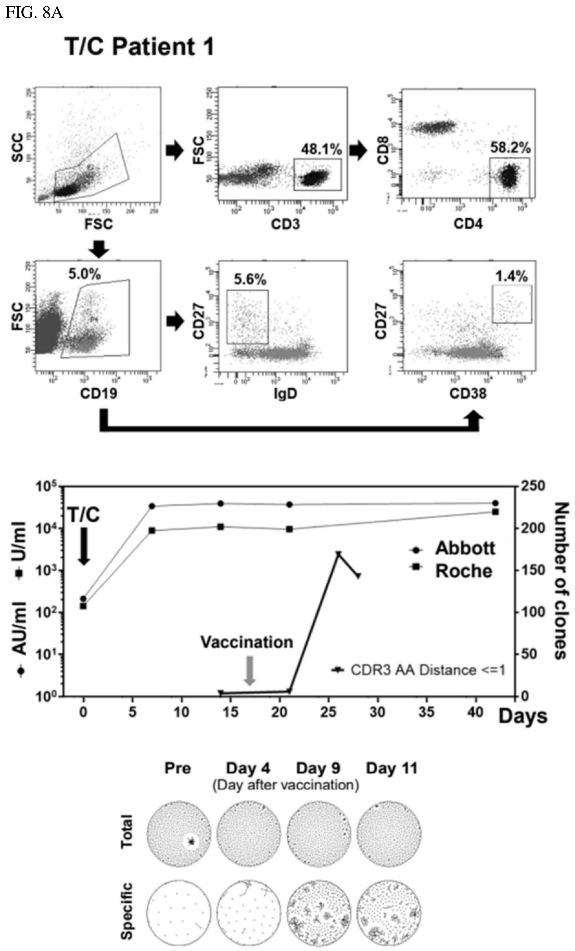

[Fig. 8A] Fig. 8A shows a temporal change in the number, frequency, and cluster of SARS-CoV-2 specific sequences after mRNA vaccination in a hematopoietic stem cell transplantation recipient to which tixagevimab/cilgavimab was administered. A recipient (T/C patient 1) on Day 338 after cord blood transplantation was administered with tixagevimab/cilgavimab (T/C), and then administered with an mRNA vaccine (at the time point indicated by the arrow of Vaccination). Immune cell reconstitution of helper T cells (CD3+CD4+), class-switched B cells (CD19+CD27+IgD-), plasma blasts (CD19+CD27+CD38+) was confirmed by FACS analysis at the time of vaccination. The anti-SARS-CoV-2 antibody titer was measured using Abbott SARS-CoV-2 IgG II Quant kit (Abbott) and Roche Elecsys anti-SARS-CoV-2 S kit (Roche). SARS-CoV-2 specific sequences were measured before vaccination and on Days 4, 9, and 11 after vaccination. The graph of the triangle dots shows the number of clones (CDR3AA Distance <= 1). T/C: tixagevimab/cilgavimab.

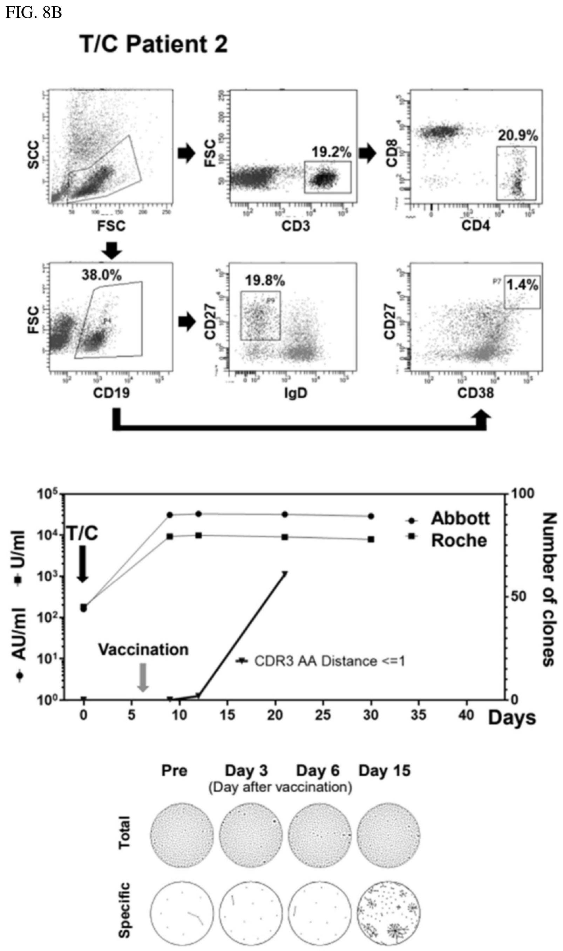

[Fig. 8B] Fig. 8B shows a temporal change in the number, frequency, and cluster of SARS-CoV-2 specific sequences after mRNA vaccination in a hematopoietic stem cell transplantation recipient to which tixagevimab/cilgavimab was administered. In T/C patient 2, a recipient was administered with tixagevimab/cilgavimab on Day 212 after unrelated bone marrow transplantation, and then vaccinated with an mRNA vaccine (the time point indicated by the arrow of Vaccination). SARS-CoV-2 specific sequences were measured before vaccination and Days 3, 6, and 15 after vaccination. The graph of the triangle dots shows the number of clones (CDR3AA Distance <= 1). T/C: tixagevimab/cilgavimab.

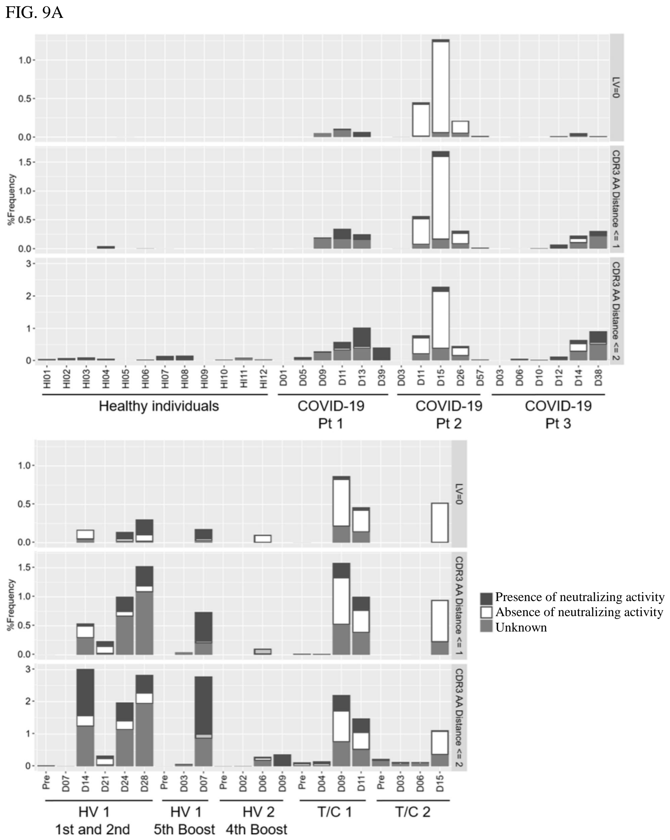

[Fig. 9A] Fig. 9A shows characteristics of SARS-CoV-2 specific sequences induced by COVID-19 infection or vaccination. Neutralizing function and non-neutralizing function of SARS-CoV-2 specific sequences detected in healthy individuals, COVID-19 patients, and vaccinated healthy volunteers. Frequencies of the SARS-CoV-2 unique sequences with neutralizing activity (Neut+) or without neutralizing activity (Neut-) detected upon vaccination of healthy individuals (HI1 to HI12), COVID-19 patients (Pt1, Pt2, and Pt3), a vaccinated healthy volunteer (HV1), booster vaccinated healthy volunteers (HV1 and HV2), and hematopoietic stem cell transplantation patients vaccinated with tixagevimab/cilgavimab (TC1 and TC2) are shown. The frequencies of SARS-CoV-2 specific sequences (LV = 0, 1, and 2, respectively) with different CDR3 amino acid distances are shown.

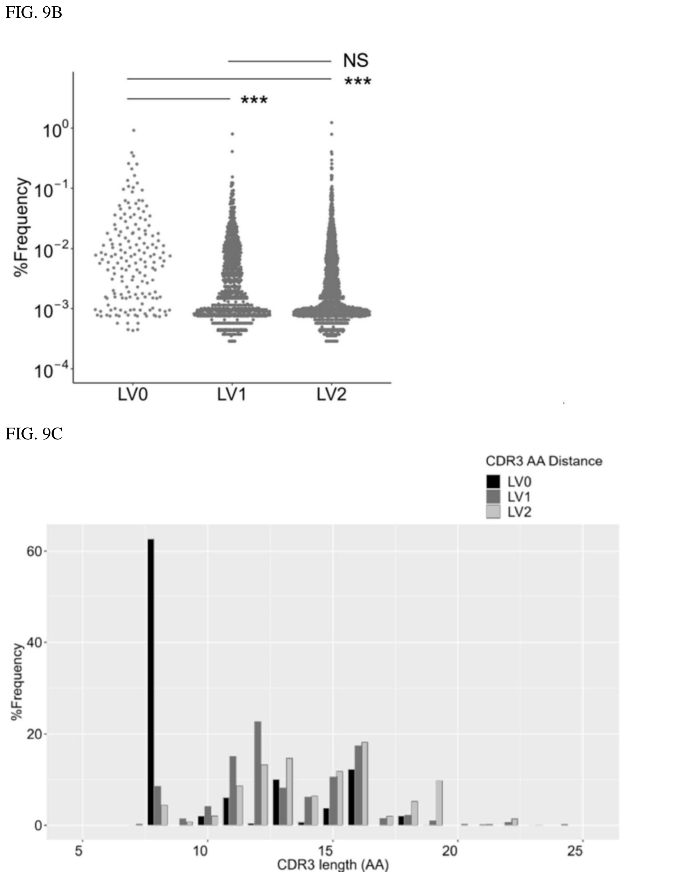

[Fig. 9B] Fig. 9B shows frequencies in local samples of SARS-CoV-2 specific sequences with different CDR3 amino acid distances. Each dot indicates SARS-CoV-2 specific sequences detected in each sample of vaccinated healthy volunteers (n = 2), COVID-19 patients (n = 3), booster vaccinated volunteers (n = 2), and vaccinated transplantation recipients (n = 2). ***: p < 0.001, NS: not significant, by Kruskal-Wallis test and Dunn-Bonferroni post hoc test.
[Fig. 9C] Fig. 9C shows a distribution of CDR3 amino acid lengths of SARS-CoV-2 specific sequences having different CDR3 amino acid distances. The percentage frequency of different SARS-CoV-2 specific sequences of CDR3 length detected in this study is shown.

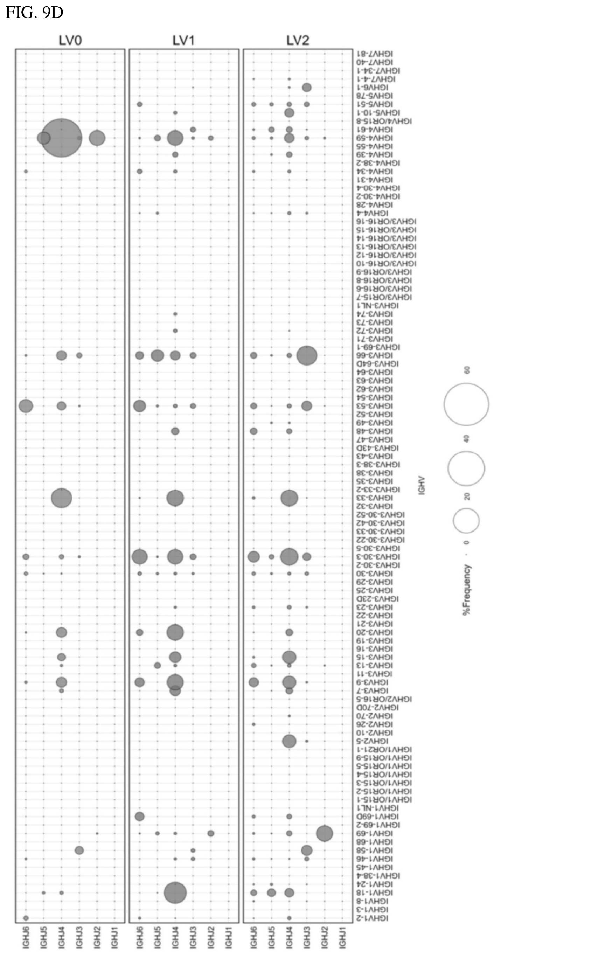

[Fig. 9D] Fig. 9D shows VJ use frequencies of SARS-CoV-2 specific sequences having different CDR3 amino acid distances. The percent of VJ use frequencies of SARS-CoV-2 specific sequences detected in this study is shown in a bubble chart. The X-axis indicates the IGHV gene, the Y-axis indicates the IGHJ gene, and the size of the bubble indicates the use frequency.

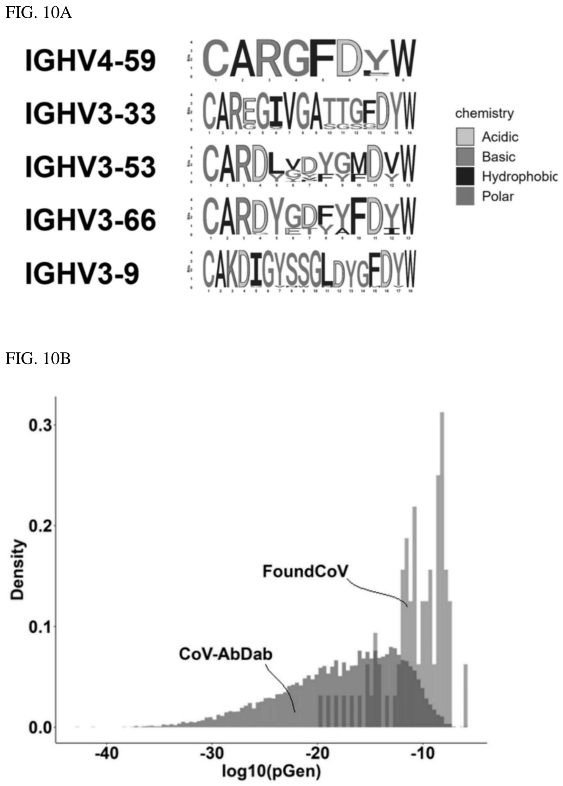

[Fig. 10A] Fig. 10A shows characteristics of SARS-CoV-2 specific sequences induced by SARS-CoV-2 infection or vaccination. Alignment of SARS-CoV-2 specific sequences of infected individuals and vaccinated individuals. The CDR3 amino acid sequence (Tables 3A and 3B, SARS-COV-2 specific sequences of infected and vaccinated individuals (completely identical)) with IGHV4-59, IGHV3-33, IGHV3-53, IGHV3-66, IGHV3-9, frequent in infected individuals and vaccinated individuals, was aligned in Clustal Omega, and the sequence logo was drawn in the ggseqlogo package.
[Fig. 10B] Fig. 10B shows characteristics of SARS-CoV-2 specific sequences induced by SARS-CoV-2 infection or vaccination. Generation probability (pGen) values were compared. A histogram of pGen values for 8,977 SARS-CoV-2 specific sequences in the CoV-AbDab database and 86 completely identical SARS-CoV-2 specific sequences (FoundCoV) detected from infected individuals and vaccinated individuals is shown. The pGen value was calculated from the CDR3 amino acid sequence of each sequence using the OLGA package. A sequence having a high pGen value has a high occurrence probability, and a sequence having a low pGen value is a sequence that rarely occurs.

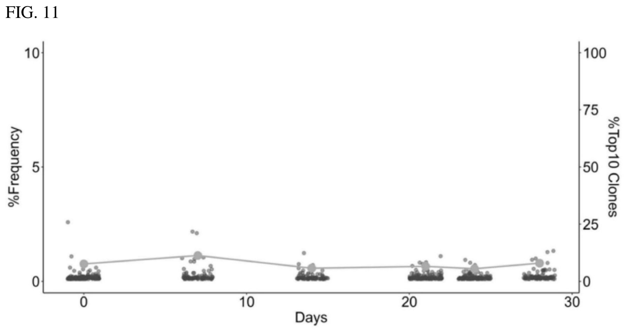

[Fig. 11] Fig. 11 shows occupancy frequencies of dominant individual clones and top 10 clones in vaccinated healthy volunteers. Individual clones with an occupancy frequency of more than 0.1% in vaccinated healthy volunteers are indicated by dots. The sum of the occupancy frequencies of the top 10 clones is indicated by a polygonal line.

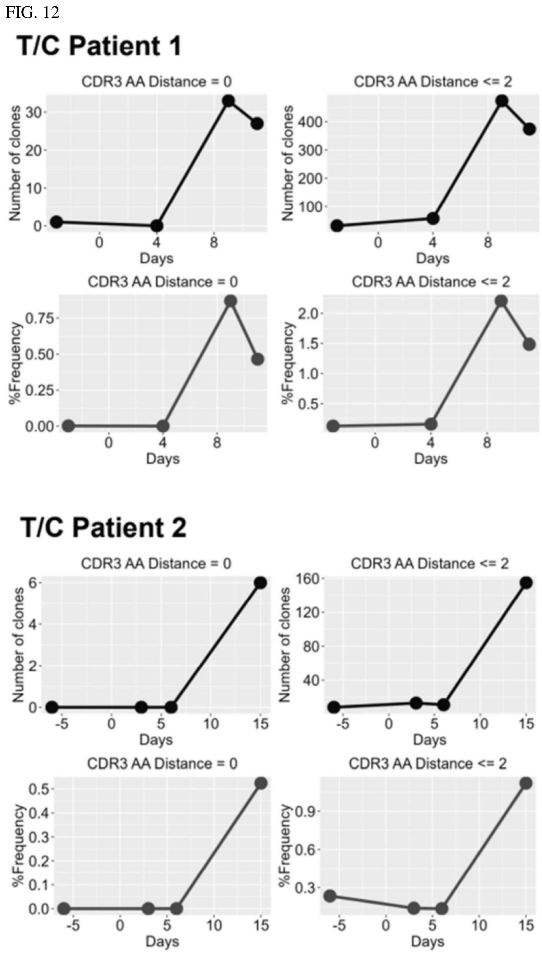

[Fig. 12] Fig. 12 shows a temporal change in the number and frequency of SARS-CoV-2 specific sequences after mRNA vaccination in a hematopoietic stem cell transplantation recipient to which a SARS-CoV-2 antibody was administered. The number of clones (top) and percent frequency (bottom) of SARS-CoV-2 specific sequences are plotted with respect to time after vaccination (Days).

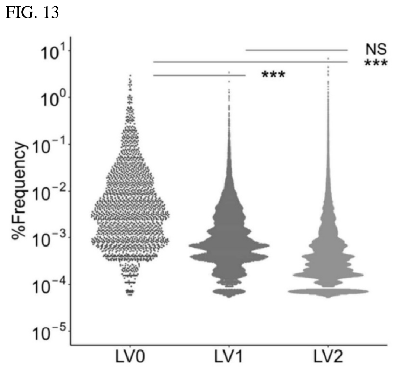

[Fig. 13] Fig. 13 shows the frequencies of SARS-CoV-2 specific sequences in the OAS database. Among the IGHG sequences in the OAS database reported from 2011 to 2020, the frequency ratios in individual samples having V and J genes that are the same as the SARS-CoV-2 specific sequence of CoV-AbDab, and having an amino acid sequence edit distance of CDR3 that is different from 0 (LV0), 1 (LV1), or 2 (LV2) were shown. Each dot indicates each SARS-CoV-2 specific sequence detected in the OAS database. ***p < 0.001, NS: not significant by Kruskal-Wallis test and Dunn-Bonferroni post hoc test.

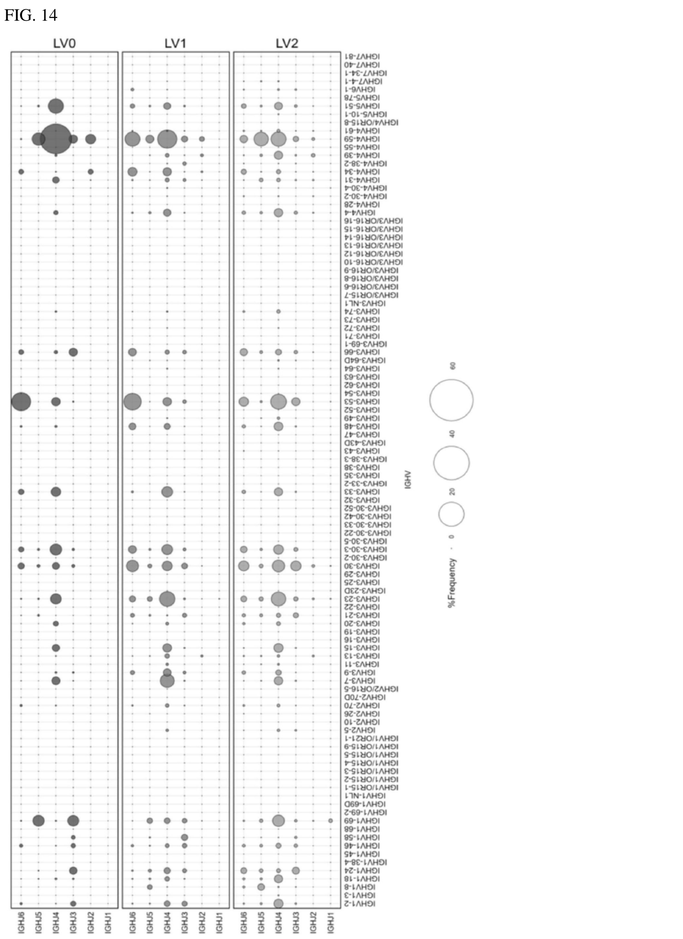

[Fig. 14] Fig. 14 shows a VJ use rate of SARS-CoV-2 specific sequences in the OAS database. The percentage frequencies of SARS-CoV-2 specific sequences with different CDR3 amino acid edit distances were displayed in different bubble charts. The X-axis indicates the IGHV gene, the Y-axis indicates the IGHJ gene, and the size of the bubble indicates the percentage of use frequency.

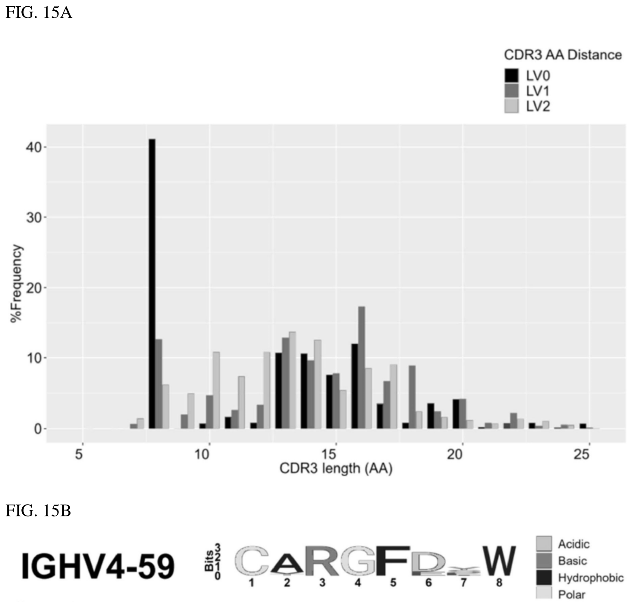

[Fig. 15A] Fig. 15A shows characteristics of the SARS-CoV-2 specific sequence. Distribution of CDR3 amino acid lengths of SARS-CoV-2 specific sequences with different CDR3 amino acid distances in the OAS database. The percentage frequency (read abundancy) of SARS-CoV-2 specific sequences with different CDR3 amino acid lengths reported in the OAS database from 2011 to 2020. SARS-CoV-2 specific sequences with different edit distances for CDR3 (LV0, LV1, LV2) were shown in separate bar graphs.
[Fig. 15B] Fig. 15B shows alignment of the SARS-CoV-2 specific sequence with IGHV4-59 and CDR3 sequence of 8 amino acids. Sequence logos were generated with the ggseqlogo package.

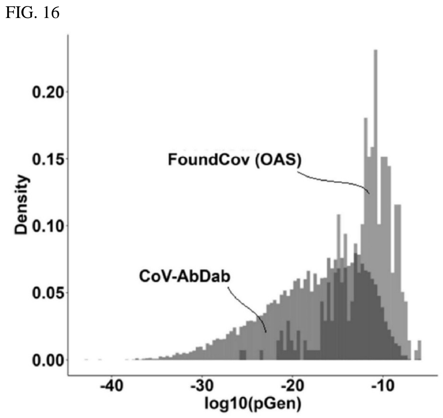

[Fig. 16] Fig. 16 shows comparison of generation probability (pGen) values of SARS-CoV-2 specific sequences. The pGen values of 8,977 SARS-CoV-2 specific sequences in the CoV-Ab database (CoV-AbDab) and 372 completely identical SARS-CoV-2 specific sequences detected in the OAS database (FoundCoV (OAS)) are shown in a histogram. The X-axis shows the density and the Y-axis shows the logarithmic value of pGen (log10).

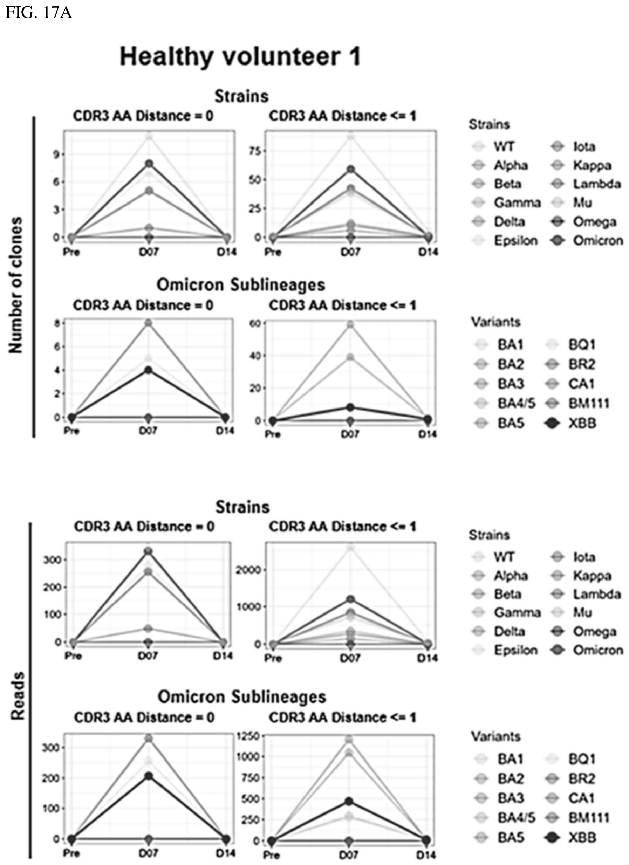

[Fig. 17A] Fig. 17A shows that an increase in antibody sequence binding to various strains (mutant strain, variant of omicron strain) in the body of the same test subject caused by vaccination with an mRNA vaccine against SARS-CoV-2 was confirmed.

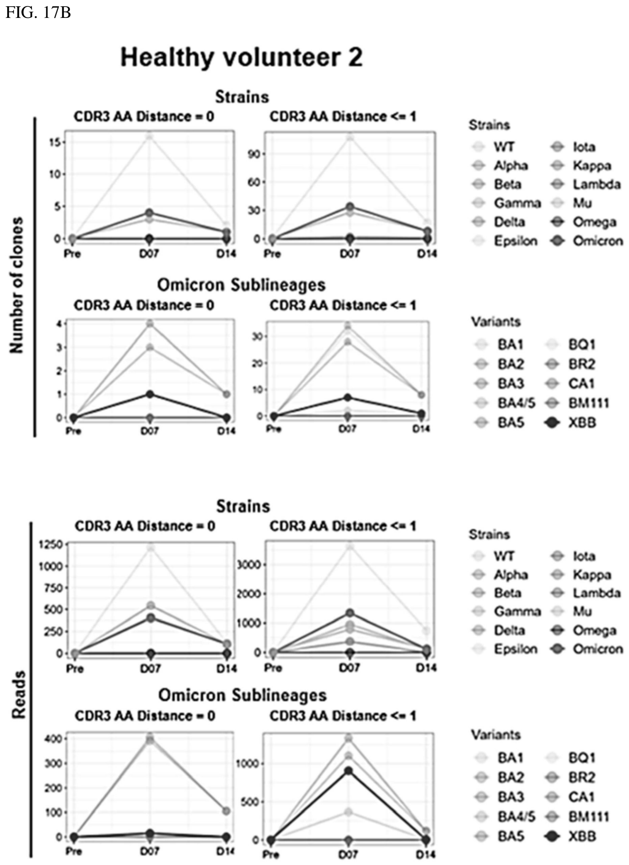

[Fig. 17B] Fig. 17B shows that an increase in antibody sequence binding to various strains (mutant strain, variant of omicron strain) in the body of the same test subject caused by vaccination with an mRNA vaccine against SARS-CoV-2 was confirmed.

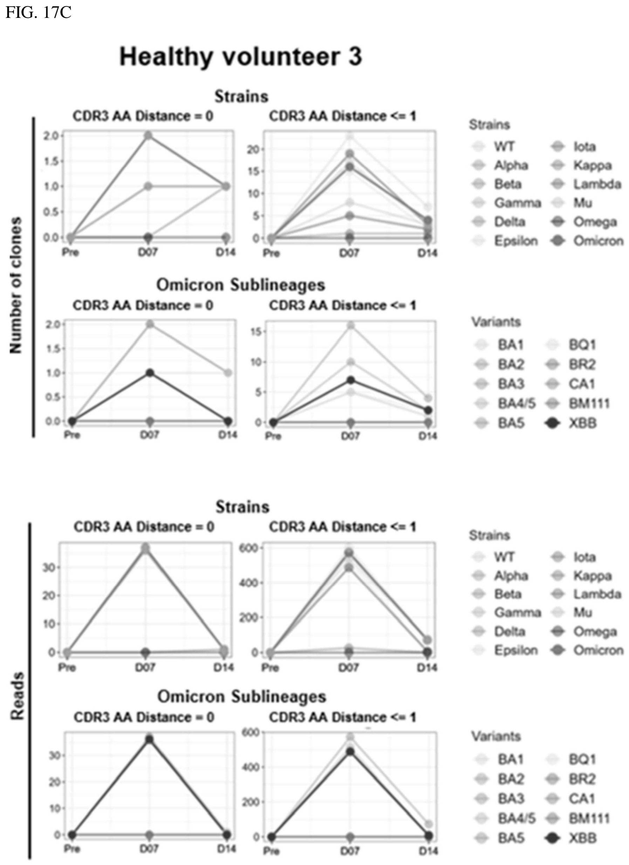

[Fig. 17C] Fig. 17C shows that an increase in antibody sequence binding to various strains (mutant strain, variant of omicron strain) in the body of the same test subject caused by vaccination with an mRNA vaccine against SARS-CoV-2 was confirmed.

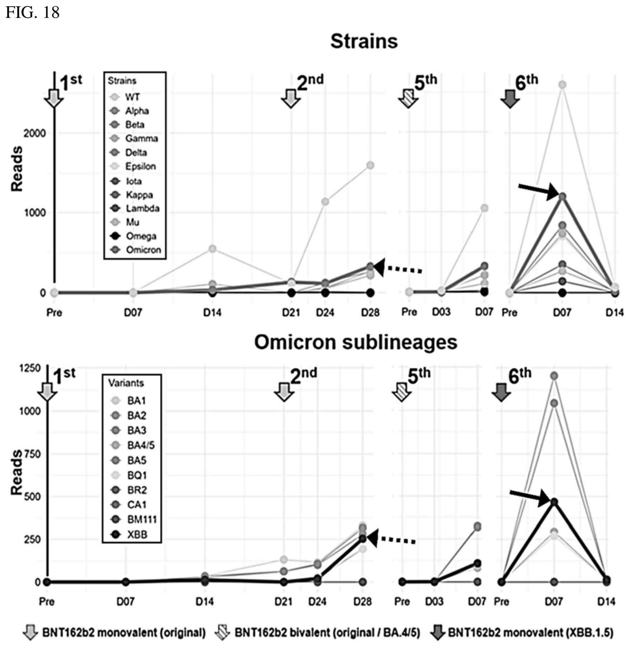

[Fig. 18] Fig. 18 shows that it was confirmed that the antibody sequences binding to different strains depending on the vaccine increased by vaccinating the same subject with an mRNA vaccine against SARS-CoV-2 of different designs of target strains.

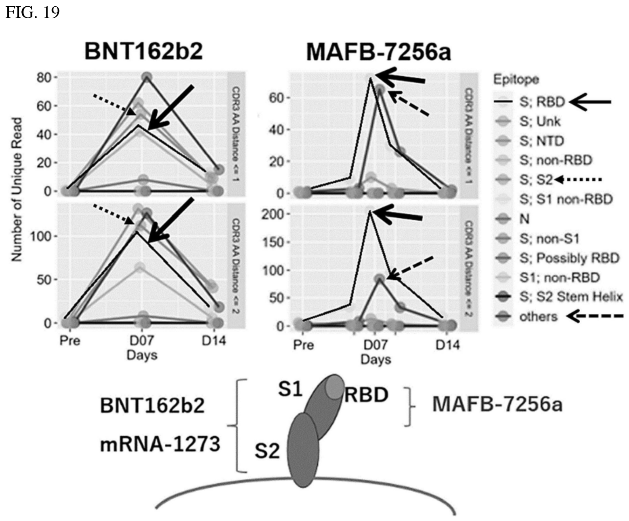

[Fig. 19] Fig. 19 shows that it was confirmed that the antibody sequences binding to different epitopes depending on the vaccine increased by vaccination with an mRNA vaccine against SARS-CoV-2 of different designs of target epitopes.

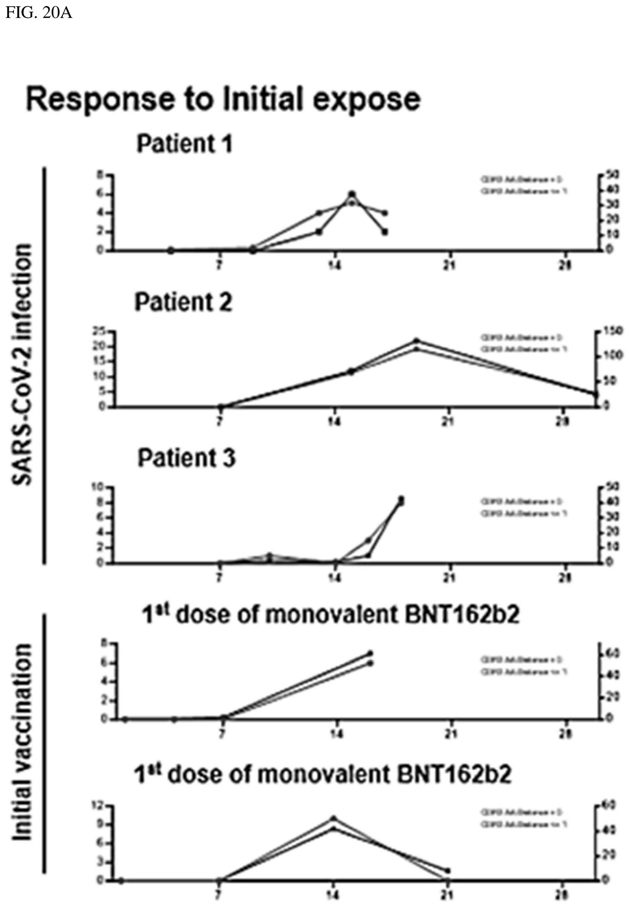

[Fig. 20A] Fig. 20A shows that, after infection or initial vaccination, the sequence identical to the database in BCR repertoire (an antibody sequence having a Levenshtein distance in which the amino acid difference of CDR3 is 0 and an antibody sequence having a Levenshtein distance of 1 or less) increased in about 2 weeks.

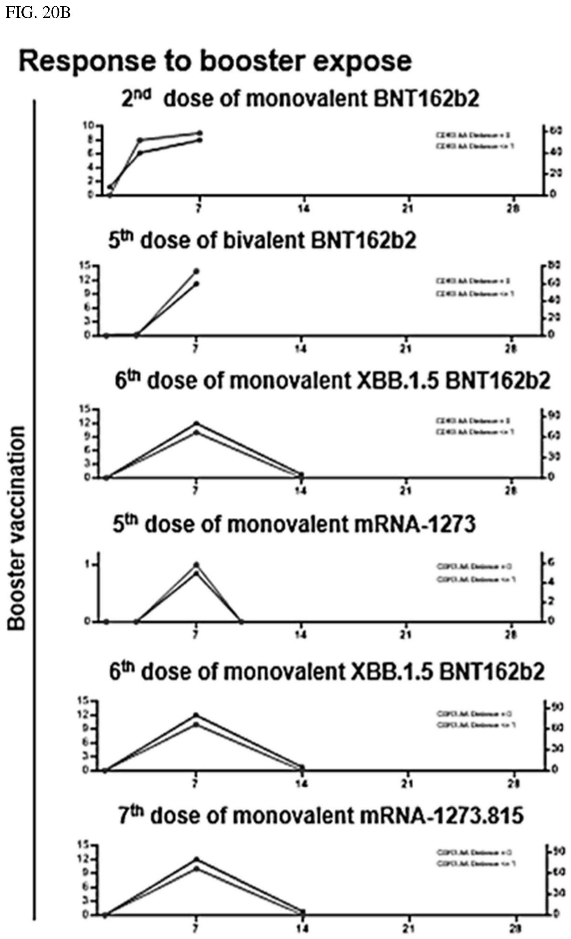

[Fig. 20B] Fig. 20B shows that, after booster vaccination, the sequence identical to the database in BCR repertoire (an antibody sequence having a Levenshtein distance in which the amino acid difference of CDR3 is 0 and an antibody sequence having a Levenshtein distance of 1 or less) increased in about 1 week.

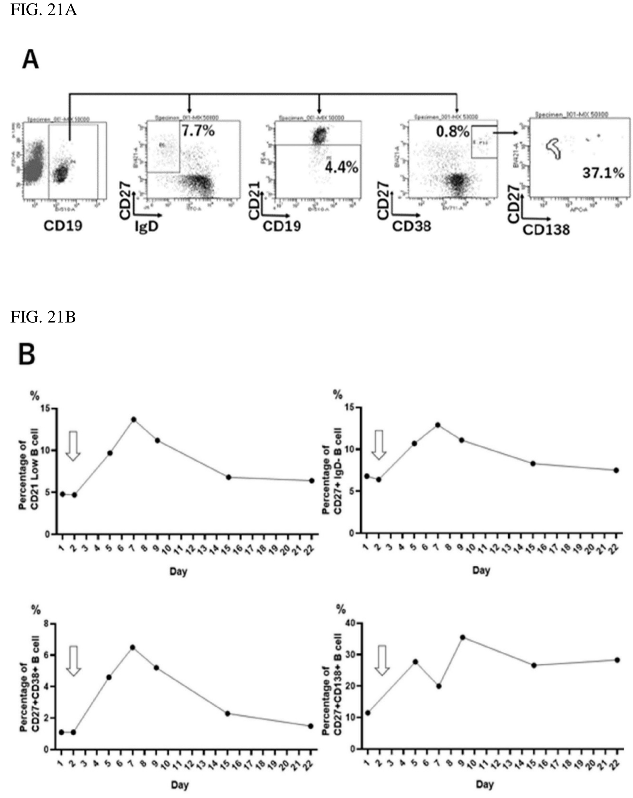

[Fig. 21A] Fig. 21A shows description of subset classification of B cells.
[Fig. 21B] Fig. 21B shows that activated B cells increased in about 1 week after cancer immunotherapy (booster immunization).

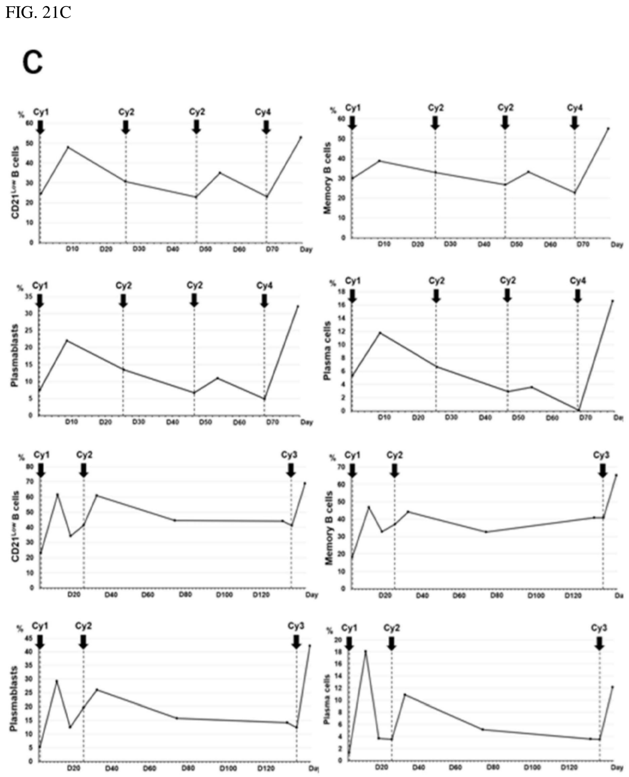

[Fig. 21C] Fig. 21C shows that activated B cells increased in about 1 week after cancer immunotherapy (booster immunization).

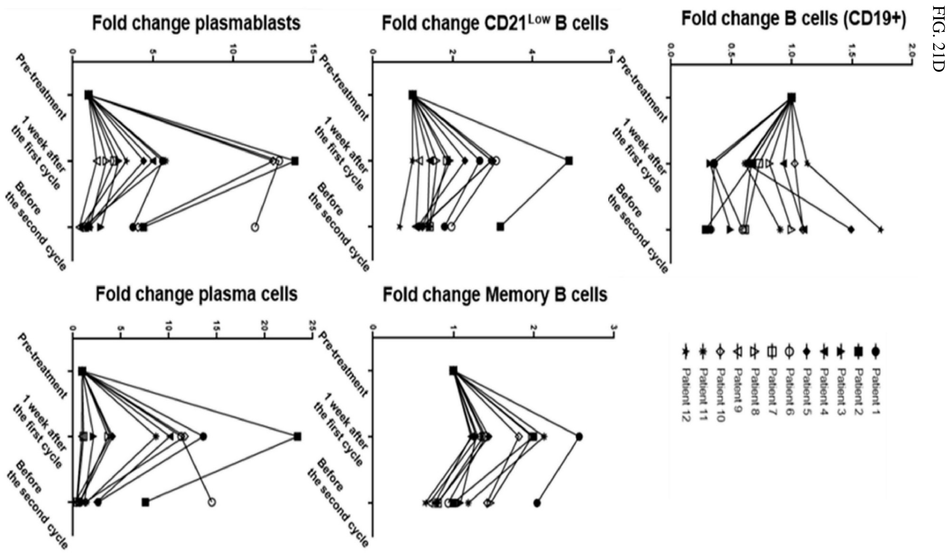

[Fig. 21D] Fig. 21D shows that activated B cells increased in about 1 week after cancer immunotherapy (booster immunization) and a larger increase in activated B cells was further observed in cases (Patients 1, 2, 6, 10, and 11) who developed irAE.

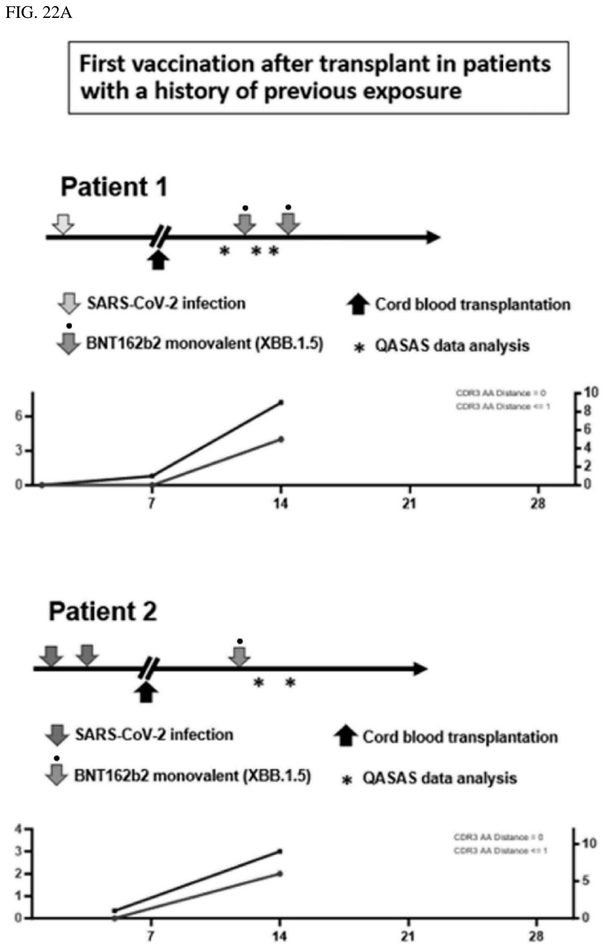

[Fig. 22A] Fig. 22A shows that the sequence identical to the database in BCR repertoire (an antibody sequence having a Levenshtein distance in which the amino acid difference of CDR3 is 0 and an antibody sequence having a Levenshtein distance of 1 or less) increased by a first antigen stimulation (vaccination) after hematopoietic stem cell transplantation, in 2 weeks from the first antigen stimulation.

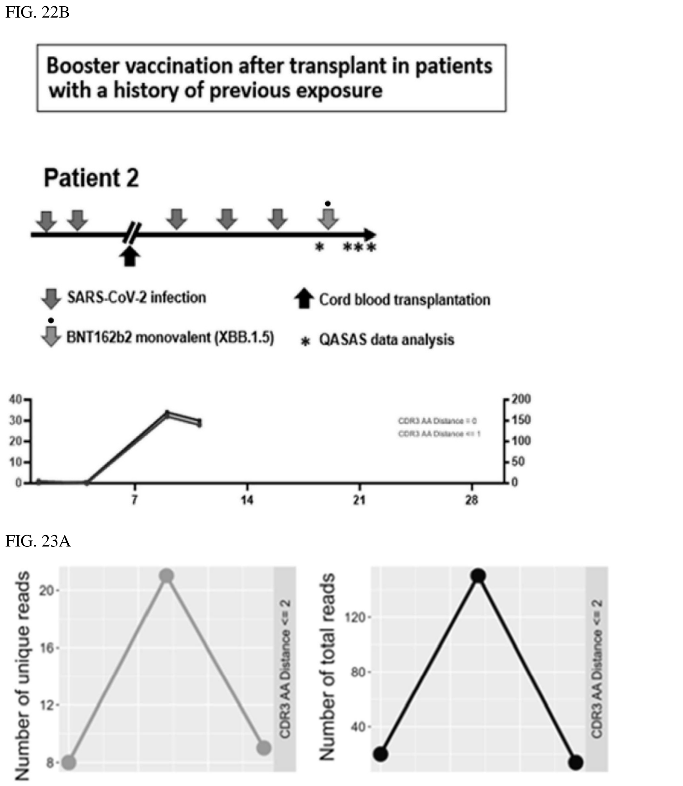

[Fig. 22B] Fig. 22B shows that the sequence identical to the database in BCR repertoire (an antibody sequence having a Levenshtein distance in which the amino acid difference of CDR3 is 0 and an antibody sequence having a Levenshtein distance of 1 or less) increased by a second or subsequent antigen stimulation (vaccination) after hematopoietic stem cell transplantation, in 1 week from the second or subsequent antigen stimulation.
[Fig. 23A] Fig. 23A shows that, after the influenza inactivated vaccination, the sequence identical to the database (about 1300 sequences independently collected by citation from the article are listed) in the BCR repertoire increased in about 1 week.

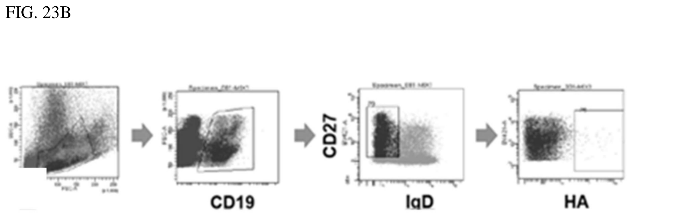

[Fig. 23B] Fig. 23B shows an in vitro experiment related to sequence collection for expansion of the database used in Fig. 23A.

### Description of Embodiments

A method for evaluating an immune reaction of the present invention is a method for evaluating an immune reaction to a specific antigen using an antigen receptor repertoire analysis. Hereinafter, the method for evaluating an immune reaction of the present invention may be described as "Quantification of Antigen-specific Antibody Sequence (QASAS) method". The outline of the method for evaluating an immune reaction of the present invention is shown in Fig. 1.

The method for evaluating an immune reaction of the present invention includes: step A of preparing repertoire data [corresponding to Fig. 1(ii)] including a group of sequences of antigen recognition sites in immune cell receptors (antigen receptors), the repertoire data being acquired from a specimen of a subject [corresponding to Fig. 1(i)]; and step B of collating a database [corresponding to Fig. 1(iii)] including a group of sequences of antigen recognition sites in immune cell receptors for a specific antigen with the repertoire data [corresponding to Fig. 1(ii)] to detect, from the repertoire data [corresponding to Fig. 1(ii)], a sequence which is identical to the sequence included in the database [corresponding to Fig. 1(iii)] at a level of amino acid sequences or a sequence which is identical to the sequence included in the database at a portion excluding two or less amino acid residues [corresponding to "Sequence identical to database" in Fig. 1; in the following, a sequence which is identical at a level of amino acid sequences (i.e., a completely identical sequence) or a sequence which is identical at a portion excluding two or less amino acid residues (a similar sequence including one or two mismatches) are also simply referred to as "identical sequence"]. As a result, from many sequences included in the repertoire data (ii), a sequence immunologically reacted in the living body of the subject at a collection time of the specimen (i.e., reacted to "Antigen stimulation" in Fig. 1) [corresponding to "Sequence identical to database" in Fig. 1] can be specified. As described above, in the method of the present invention, since a sequence showing an immunologically relevant reaction, i.e., a sequence truly capable of reacting with an antigen can be specified from a large number of sequences listed in the repertoire data (ii) and the database (iii), the immune reaction can be evaluated not by the level of protein expression as in the antibody titer measurement in the conventional ELISA, but by the level of mRNA expression.

The method for evaluating an immune reaction of the present invention can further include step C of deriving the number [corresponding to "Number of identical sequences (clones)" in Fig. 1] and/or frequency [corresponding to "Proportion of identical sequences" in Fig. 1] of sequences detected in the step B in the repertoire data. This makes it possible to determine the degree of immune reaction.

The method for evaluating an immune reaction of the present invention can further include step D of confirming a temporal change in the number and/or frequency of sequences identical to the database [i.e., corresponding to the transition of "Number of identical sequences (clones)" or "Proportion of identical sequences" with respect to the "Day" axis in Fig. 1]. This makes it possible to investigate the transition of the immune reaction for each collection time of the specimen. Note that the antibody protein remains in the body for a long period of time since it takes time to reach a detectable level from the immune reaction and the half-life is also long, whereas mRNA does not remain in the body for a long period of time since it quickly reaches a detectable level after the immune reaction and the half-life is short. Therefore, as shown in the transition of "Number of identical sequences (clones)" or "Proportion of identical sequences" in Fig. 1, the immune reaction can be promptly detected on the basis of the mRNA level, and the humoral immunity activity can be grasped in real time.

Forms according to the purpose of application of the method of the present invention include the following.
Form [a] Evaluation of scientific validity of vaccine
Case a1: Ability to produce antibody with neutralizing activity
Case a2: Type of strain (mutant strain, variant) of target antigen
Case a3: Type of target epitope
Case a4: Ability to produce antibody whose antigen recognition site is completely identical to that of antibody by adaptive immunity
Form [b] Evaluation of immune reaction of vaccine
Case b1: Evaluation of immune reaction of vaccine during retaining antibody by administration of antibody drug
Case b2: Evaluation of immune reaction of vaccine during retaining antibody by other vaccination
Case b3: Evaluation of immune reaction of vaccine during retaining antibody by infection
Case b4: Evaluation of restoration of immune reaction by vaccination after immunosuppressive treatment
Form [c] Diagnosis of infectious disease
Case c1: Specifying of pathogen
Case c2: Determination of suitability of existing vaccine compatible with new pathogen
Form [d] Evaluation of cancer immunity
Case d1: Determination of response to cancer immunotherapy
Case d2: Identification of true neoantigen
Form [e] Evaluation of reaction to autoantigen
Case e1: Diagnosis of onset of autoimmune disease
Case e2: Diagnosis of disease condition of autoimmune disease
Case e3: Evaluation of side effect after administration
Form [f] Use as exhaustive biosensor

Hereinafter, the method for evaluating an immune reaction of the present invention will be described in detail.

### 1. Step A

In step A, repertoire data [corresponding to Fig. 1(i)] of sequences of antigen recognition sites in immune cell receptors which is acquired from a specimen of a subject is prepared.

### 1-1. Repertoire data

The repertoire is a total (immune repertoire) of time-variable adaptive immunity receptors possessed by a subject.

A biological sample to be a repertoire data source is not particularly limited as long as it is a sample containing immune cells, and examples thereof include body fluid samples such as blood and lymph, and homogenate fluids of tissues, preferably include body fluid samples, and more preferably include blood (still more preferably peripheral blood mononuclear cells).

The repertoire data includes sequences of antigen recognition sites in immune cell receptors. As the immune cell, a B cell or a T cell can be selected. Preferred examples of the case of selecting a B cell as the immune cell include Forms [a] to [c], [e], and [f]. Preferred examples of the case of selecting a T cell as the immune cell include Forms [d] and [f].

In the repertoire data used in the present invention, an immune cell from which the sequences of the antigen recognition sites are derived includes an immune cell specific to a specific antigen, but also includes an immune cell that is not specific to a specific antigen at random, and no distinction is made therebetween. That is, in the repertoire data used in the present invention, an immune cell from which the sequences of the antigen recognition sites are derived is not an immune cell specific to a specific antigen which is narrowed down from all immune cells obtained from the biological sample described above, and typically, can include all immune cells (e.g., all B cells or all T cells that can be specified from peripheral blood mononuclear cells) that can be specified from the biological sample described above.

The repertoire data may include information acquired from one subject or information acquired from a plurality of subjects having a common subject attribute.

The sequence of the antigen recognition site in the immune cell receptor includes a gene sequence that determines the clonotype of the immune cell. The gene sequence may be a sequence that forms a complementarity determining region by rearrangement. Specifically, examples of the gene sequence include sequences of V segments and J segments of a TCRα chain, a TCRγ chain, and an immunoglobulin L chain, and sequences of V segments, D segments, and J segments of a TCRβ chain, a TCRδ chain, and an immunoglobulin H chain. The complementarity determining region may be at least one of CDR1, CDR2, and CDR3, and preferably includes at least CDR3.

The repertoire data may be acquired by a repertoire analysis method. The repertoire analysis method is known, and a sequence (gene sequence) of an antigen recognition site in an immune cell receptor is determined from a biological sample of a subject by next generation sequencing. Typically, the repertoire data can be acquired by extracting the entire RNA from a biological sample of a subject to synthesize cDNA, nucleic acid amplifying a gene sequence of an immune cell receptor, determining a sequence on a large scale by next generation sequencing, and assigning a sequence (gene sequence) region of an antigen recognition site. In nucleic acid amplification, it is preferable to uniformly amplify the gene sequence of the immune cell receptor without applying bias (unbiased gene amplification), and the design of a multiplex primer panel for such unbiased gene amplification can also be selected on the basis of known techniques.

The number of sequences included in the repertoire data is not particularly limited, and may be determined according to the analysis scale of one time of the next generation sequencing used, the number of subjects who provided the repertoire data, and the like. Specifically, the number of sequences included in the repertoire data is, for example, 100,000 to 10,000,000, 300,000 to 5,000,000, or 500,000 to 3,000,000 in terms of the number of immune cell receptors.

Note that the repertoire analysis method that can be applied in the present invention includes not only bulk analysis for analyzing gene expression of immune cells at a cell population level, but also single cell analysis for analyzing gene expression of immune cells in individual cell units.

### 1-2. Subject

The subject is not particularly limited as long as the subject requires evaluation of an immune reaction, and can be set according to the application purpose of the method of the present invention. Specific examples of the subject include a subject exposed to a specific antigen, a subject subjected to cancer immunotherapy, and a subject suffering from an autoimmune disease. Examples of the biological species of the subject include humans and non-human animals (although not particularly limited, for example, mice, rats, zebrafish, and the like).

### 1-2-1. Subject exposed to specific antigen

The exposure to the specific antigen is not particularly limited as long as it is an event that leads to activation of an immune reaction, and examples thereof include vaccination with a vaccine and infection.

### 1-2-1-1. Subject vaccinated with vaccine as specific antigen

As a subject exposed to a specific antigen, a subject vaccinated with a vaccine as a specific antigen can be selected. In this case, as the evaluation of the immune reaction of the vaccine, not only immune reactivity to the vaccine is confirmed by specifying the sequence that reacted immunologically to the vaccine in the living body of the subject at the collection time of the specimen, but also the scientific validity (e.g., whether the vaccine exerts a predetermined function suitable for the design, and the like) of the vaccine is evaluated (including prediction). Examples of a case where the subject is selected include form [a], Form [b], and Form [f].

Since the immune reaction by the vaccine is slower than the infection, the immune reaction to a specific antigen cannot be confirmed even using the conventional repertoire analysis. Since the method of the present invention can selectively specify an immune reaction to a specific antigen from the repertoire data, the method is highly useful in that an immune reaction to a specific antigen can be effectively specified even in the case of the immune reaction by the vaccine.

The type of vaccine is not particularly limited, and specific examples thereof include nucleic acid vaccines (mRNA vaccine, DNA vaccine), viral vector vaccines, recombinant protein vaccines, inactivated vaccines, and live vaccines. The vaccine preferably includes a vaccine designed against a pathogen described later, and preferably includes a vaccine designed against the virus described later, more preferably a coronavirus or influenza virus (coronavirus vaccine or influenza virus vaccine).

In the method of the present invention, it is possible to specify an immune reaction to a specific antigen at the level of the sequence of the complementarity determining region of the antibody, and it is also possible to specify the type of strain (mutant strain, variant) or antigen information such as an epitope (antibody binding site) from the sequence information of the complementarity determining region of the antibody. Therefore, it is possible to confirm, from antigen information such as an epitope linked to the sequence of the complementarity determining region in the database, what type of strain (mutant strain, variant) the antibody produced by a vaccine containing a design drawing (genetic information) of a specific antigen as an active ingredient and whether the antibody has the ability to recognize an epitope, like a nucleic acid vaccine. From such a viewpoint, a suitable example of the type of vaccine is a nucleic acid vaccine (mRNA vaccine, DNA vaccine), and more preferably an RNA vaccine (mRNA vaccine).

### 1-2-1-2. Subject infected with specific antigen

As a subject exposed to a specific antigen, a subject infected with the specific antigen can be selected. In this case, an immune reaction in a specific infectious disease can be evaluated. Examples of a case where the subject is selected include Form [c] and Form [f].

The infection is not particularly limited as long as it is an infection caused by a specific antigen as a pathogen, and specific examples thereof include an infection caused by an antigen organism such as a virus, a bacterium, or a fungus as a pathogen.

The virus is not particularly limited, and examples thereof include coronaviruses, influenza viruses, cytomegaloviruses, rotaviruses, herpesviruses, human immunodeficiency viruses, and dengue viruses. Examples of the coronaviruses include cold coronavirus (HCoV-229E, HCoV-OC43, HCoV-NL63, HCoV-HKU1), severe acute respiratory syndrome coronavirus (SARS-CoV), Middle East respiratory syndrome coronavirus (MERS-CoV), and novel coronavirus (SARS-CoV-2). Examples of the influenza viruses include type A (including any combination of 16 hemagglutinins (HA) H1 to H16 and 9 neuraminidases (NA) N1 to N9), type B, and type C.

The bacterium is not particularly limited, and examples thereof include Pseudomonas aeruginosa, legionella bacteria, Yersinia bacteria, Escherichia coli, Vibrio bacteria, Haemophilus influenzae, Gram-negative bacilli, and Mycobacterium tuberculosis.

The fungus is not particularly limited, and examples thereof include Candida and Aspergillus.

Note that, in the present invention, the specific antigen to be a pathogen (indicating a pathogen microorganism) includes not only an antigen for which infection is currently confirmed and species and strains are specified, but also a pathogen such as a mutant strain newly generated in the future. According to the method of the present invention when the subject is selected, since the antigen information can be specified from the sequence information related to the repertoire data with the existing database, in the diagnosis of infectious diseases, it is possible not only to specify which pathogen causes the infectious disease, but also to determine whether or not the existing vaccine can be applied to a new occurring pathogen.

### 1-2-1-3. Subject retaining another antibody at high level

The subject exposed to a specific antigen (the subject described in the above item "1-2-1-1" and the above item "1-2-1-2") may further retain another antibody at a high level at the time of specimen collection. The other antibody refers to an antibody different from an antibody generated by exposure to the specific antigen. Examples of a case where the subject is selected include Forms [a] to [c] and Form [f].

As the subject retaining another antibody at a high level, a subject administered with an antibody drug, vaccinated with a vaccine, and/or infected is exemplified. Hereinafter, the subject administered with an antibody drug is also referred to as "subject (S1)", a subject vaccinated with a vaccine is also referred to as "subject (S2)", and an infected subject is also referred to as "subject (S3)".

The subject (S1) is not particularly limited, and specific examples thereof include a subject subjected to an immunosuppressive treatment and a patient suffering from an autoimmune disease. Examples of the immunosuppressive treatment include the treatment described in item 1-2-3 described later. Examples of the autoimmune disease include diseases described in item 1-2-4 described later. The antibody drug is not particularly limited, and any antibody pharmaceutical agent can be used. As an example of the antibody drug, an antibody pharmaceutical agent to be administered to a subject subjected to an immunosuppressive treatment, for example, to counter exposure to a specific antigen is exemplified. For example, when the specific antigen is a novel coronavirus, examples of the antibody pharmaceutical agent include tixagevimab/cilgavimab, casirivimab/imdevimab, and sotrovimab. Other examples of the antibody drug include an antibody pharmaceutical agent used for treatment of autoimmune diseases. This antibody pharmaceutical agent can be appropriately selected by those skilled in the art according to the type of autoimmune disease.

The vaccine with which the subject (S2) is vaccinated is not particularly limited. However, when the subject of the above item "1-2-1-1" is further the subject (S2), the vaccine that is a condition for the subject (S2) is another vaccine different from the vaccine that is a condition for the subject of the above item "1-2-1-1". Specific examples of the other vaccine include a vaccine that produces an antibody different from the antibody produced by the vaccine (i.e., specific antigen) that is a condition for the subject of the above item "1-2-1-1", and a vaccine designed to target another antigen (an antigen other than the specific antigen).

The pathogen in the subject (S3) is not particularly limited. However, when the subject of the above item "1-2-1-2" is further the subject (S3), the pathogen that is a condition for the subject (S3) is another pathogen different (i.e., different at least at the class level of strain) from the pathogen (i.e., specific antigen) that is a condition for the subject of the above item "1-2-1-2". Specifically, the other pathogen can be appropriately selected from the pathogens listed in the above item "1-2-1-2".

Due to administration of an antibody drug, vaccination with a vaccine, or infection, another antibody (specifically, an antibody related to an antibody drug, an antibody produced in an immune reaction to a vaccine, or an antibody produced in an immune reaction to a pathogen) is present at a high level in the bodies of the subject (S1) to the subject (S3). Since the half-life of the antibody protein is long, these other antibodies are retained at a high level in the bodies of the subject (S1) to the subject (S3). In this case, even when an antibody is generated by an immune reaction to a specific antigen separately in parallel, such an antibody is masked by a large amount of other coexisting antibodies, and thus cannot be confirmed by conventional repertoire analysis or an immunological measurement method such as ELISA. Since the method of the present invention can selectively specify an immune reaction to a specific antigen from the repertoire data without being affected by the amount of antibody protein in the body, even in a subject retaining another antibody at a high level, the immune reaction can be specified as long as an immune reaction to a specific antigen occurs.

The relationship between the time of administration of an antibody drug and the time of antigen exposure is not particularly limited as long as the biological sample related to the repertoire data is acquired while the antibody related to the antibody drug is retained in the body. Therefore, within the above limits, the time of administration of an antibody drug may be any of before antigen exposure, simultaneously with antigen exposure, and after antigen exposure, and is preferably before antigen exposure.

### 1-2-2. Subject subjected to cancer immunotherapy

In the body of a subject subjected to cancer immunotherapy (a treatment method for enhancing an immune reaction to cancer originally included in the body), immune cells re-recognize a cancer antigen that has been tolerant so far. This re-recognition of immune cells against a cancer antigen (neoantigen) involves, in principle, changes in the immune environment (activation of immune reaction) similar to antigen exposure. As described above, since the method of the present invention can evaluate an immune reaction activated by exposure to an antigen, an immune reaction activated by cancer immunotherapy can also be evaluated by the same mechanism. Therefore, a subject subjected to cancer immunotherapy can be selected as the subject. Examples of a case where the subject is selected include Form [d] and Form [f]. As the specific antigen, a cancer antigen can be selected.

Examples of the cancer immunotherapy include a treatment method of removing the brake on immunity by cancer, and specific examples thereof include administration of an immune checkpoint inhibitor that activates cancer immunity by inhibiting signaling of immunosuppression. Examples of the immune checkpoint inhibitor include an anti-PD-1 antibody, an anti-PD-L1 antibody, and an anti-CTLA4 antibody.

By evaluating the immune reaction of the subject subjected to cancer immunotherapy, it is possible to determine whether or not a predetermined function of the cancer immunotherapy is exerted (i.e., the response to cancer immunotherapy). According to the method of the present invention when the subject is selected, since an antigen identical to the T cell receptor database can be specified as a clinically significant T cell receptor sequence from sequence information related to T cell receptor repertoire data of a specimen collected after cancer immunotherapy, an antigen (typically, a peptide) that binds to the T cell receptor sequence can be identified as a true neoantigen.

### 1-2-3. Subject subjected to immunosuppressive treatment

As a subject, a subject subjected to an immunosuppressive treatment can be selected. Examples of a case where the subject is selected include Case b4 of Form [b] and Form [f]. As the specific antigen, a vaccine can be selected.

The immunosuppressive treatment is not particularly limited, and examples thereof include hematopoietic stem cell transplantation and administration (chemotherapy) of B cell depletion therapy or the like. In the case of use in evaluation of an immune reaction of a subject subjected to an immunosuppressive treatment, for example, it is possible to determine whether or not the immune reaction reduced by the immunosuppressive treatment is restored by antigen stimulation (such as vaccination) after the immunosuppressive treatment.

Note that, when such a subject is selected, the subject only needs to receive the antigen stimulation with a specific antigen after the immunosuppressive treatment, and may or may not receive the stimulation with an antigen (this antigen may be the same antigen as the specific antigen, or may be an antigen different from the specific antigen) before the immunosuppressive treatment. Note that, even when the stimulation with the same antigen as the specific antigen is received before the immunosuppressive treatment, the immunity is reset by the immunosuppressive treatment. Therefore, in the method of the present invention, even when the stimulation with the same antigen as the specific antigen is received before the immunosuppressive treatment, the reaction caused by the stimulation with the specific antigen received after the immunosuppressive treatment is positioned as a primary reaction of the immune reaction (see Fig. 22A).

### 1-2-4. Subject suffering from autoimmune disease

As a subject, a subject suffering from an autoimmune disease can be selected. Examples of a case where the subject is selected include Form [e] and Form [f]. As the specific antigen, an autoantigen can be selected.

The autoimmune disease is not particularly limited, and examples thereof include myasthenia gravis (autoantigen: acetylcholine receptor), immune thrombocytopenia (ITP) (autoantigen: platelet), and Type 1 diabetes (autoantigen: β cell).

According to the method of the present invention when the subject is selected, the onset or disease condition of the autoimmune disease can be evaluated by specifying a sequence identical to a database relating to an immune cell receptor for an autoantigen that causes an autoimmune disease from sequence information relating to immune cell receptor repertoire data acquired from the test subject.

According to the method of the present invention when the subject is selected, evaluation of side effects after drug administration can also be performed. In this case, the off-target effect of the drug can be evaluated by constructing a database group for autoantigens and confirming that sequences identical to the database for the target different from the design target of the drug increase by the drug administration. For example, when SARS-CoV-2 vaccine is administered, for example, in a case where an increase in the sequence identical to the database of the myocardium, not the sequence of the database for SARS-CoV-2, among the myriad databases constituting the database group is observed by repertoire analysis, it can be evaluated that myocarditis occurs as a side effect. This makes it possible to perform screening for predicting the appearance of unexpected side effects (antibodies that react with autoantigens) for a new drug, and thus it becomes possible to develop a new drug with higher safety.

### 1-3. Acquisition time of repertoire data

The acquisition time of the repertoire data of the subject (i.e., the collection time of a biological sample serving as a repertoire data source) is not particularly limited, and can be selected from any time when the evaluation of the immune reaction is required. Preferably, a time that coincides with a time of activation of mRNA of an immune cell receptor for a specific antigen (i.e., the time of clonal expansion of immune cells in response to a specific antigen in the repertoire) is selected. The specific time of activation of mRNA does not depend on the type of the specific antigen, and is, for example, 4 to 18 days after immune stimulation with the specific antigen. More specifically, in the case of evaluating a primary reaction among immune reactions (e.g., in the case of evaluating immunity of infection, primary vaccination with a vaccine, or cancer immunotherapy), the time of activation is after antigen stimulation for the primary reaction, for example, 11 to 20 days or 11 to 18 days, preferably 12 to 16 days, more preferably 13 to 15 days, and in the case of evaluating a secondary reaction, a tertiary reaction, ..., or an n-th reaction (n is an integer representing the number of times of the immune reaction; these secondary and subsequent reactions are also collectively referred to as "secondary and subsequent reactions") (for example, in the case of evaluating booster vaccination with a vaccine or cancer immunotherapy), the time of activation is after antigen stimulation for a secondary reaction, a tertiary reaction, ..., or an n-th reaction, respectively, for example, 3 to 10 days or 4 to 10 days, preferably 5 to 9 days, and more preferably 6 to 8 days.

### 2. Step B

In step B, a database [corresponding to Fig. 1(iii)] of a sequence of an antigen recognition site for a specific antigen and the repertoire data [corresponding to Fig. 1(ii)] are collated with each other to detect, from the repertoire data, a sequence which is identical to the sequence included in the database at a level of amino acid sequences or a sequence which is identical to the sequence included in the database at a portion excluding two or less amino acid residues [corresponding to "Sequence identical to database" in Fig. 1].

### 2-1. Database

Sequences of antigen recognition sites for a specific antigen are listed in a database. The specific antigen is the same as the specific antigen described above "1-2. Subject". The sequence of the antigen recognition site is as described above as the sequence of the antigen recognition site in the above "1-1. Repertoire data".

In addition to the information of the sequence of the antigen recognition site for the specific antigen, the database may include additional information, such as an antibody sequence/nanobody sequence/variable region sequence for the specific antigen, presence or absence of neutralizing activity of the antibody, an epitope region in the specific antigen, and a species/strain (mutant strain, variant) of an antigen organism, (hereinafter, also simply referred to as "additional information").

### 2-1-1. Public database

An example of such a database is CoV-AbDab (database managed by Oxford Protein Informatics Group of Department of Statistics, The University of Oxford) as a public database. Information on β coronaviruses such as SARS-CoV-2, SARS-CoV-1, and MERS-CoV is listed in CoV-AbDab, and information on the sequence/nanobody sequence/variable region sequence of an antibody bindable to each β coronavirus, presence or absence of neutralizing activity of the antibody, an epitope region, a species/strain (mutant strain, variant) of each β coronavirus, and the like are included.

### 2-1-2. Independently created database

In the present invention, as the database, as long as the above-described information is included, a publicly available database may be used, or a database created by independently collecting data for each specific antigen may be used. The sequence of the antigen recognition site for a specific antigen to be listed in the database can be appropriately acquired using a known method. Examples of the data collection method include the following methods [1] to [3]. In order to collect the database, any one of the methods shown in [1] to [3] may be used, or two or more methods may be used in combination.
[1] Collection from existing literature and/or public database
[2] Sequence collection by in vitro experiments
[3] Sequence collection by in silico

In the method [1] described above, a sequence of a known antigen recognition site for a specific antigen is selected from existing literature and/or a public database, and known additional information is further associated as necessary, whereby information to be stored in the database can be edited.

The method [2] described above includes, for example, a step of preparing a labeled protein in which a protein synthesized from an antigen sequence is labeled, a step of sorting cells that bind to the labeled protein in an immune cell population, and a step of acquiring a sequence of the sorted cells as information to be collected in a database.

In the method [3] described above, the sequence of the antigen recognition site in the immune cell receptor can be collected using the binding prediction of the antigen/antigen receptor using machine learning. As the binding prediction, antigen/epitope binding analysis using a protein language model (e.g., Bioinformatics, Volume 37, Issue Supplement_1, July 2021, Pages i237-i244, and Bioinformatics, Volume 39, Issue 1, January 2023, btac820), protein structural analysis using a diffusion model (e.g., Nature. 2024 May 8. doi: 10.1038/s41586-024-07487-w), binding analysis thereof, and the like are known.

In the method in which the above [2] and [3] are combined, immune cells bindable to a specific antigen are acquired from a population consisting of objects to be examined in which an immune reaction by the specific antigen has occurred, and a sequence of an antigen recognition site in an immune cell receptor can be collected by repertoire analysis. The method in which the above [2] and [3] are combined preferably includes the following steps:
· a step of acquiring, from each specimen of the objects to be examined, repertoire data in time series at a time Tₑₓ of activation of mRNA of an immune cell receptor for the specific antigen, a time T_{bf} before the time of activation and a time T_{af} after the time of activation, the repertoire data including a group of sequences of antigen recognition sites in the immune cell receptors; and
· a step of selecting a sequence of an antigen recognition site in an immune cell receptor at which proliferation is observed at the time Tₑₓ of activation as information to be collected in the database.

A conceptual diagram of an example of creating a database by this method is shown in Fig. 2. As described in the above "1-3. Acquisition time of repertoire data", it has been found that the time of activation of mRNA of an immune cell receptor (i.e., the time of clonal expansion of immune cells in response to a specific antigen in the repertoire) arrives at a predetermined period after stimulation with the specific antigen. Therefore, it is possible to acquire repertoire data of the immune cell receptors in time series including before the activation (T_{bf}: before clonal expansion), the time of activation (Tₑₓ: clonal expansion phase), and after the activation (T_{af}: after clonal expansion: shrinkage of expanded clone) based on the predetermined period (clonal expansion phase) after antigen stimulation described in the above "1-3. Acquisition time of repertoire data". In the repertoire analysis in bulk, enormous data (for example, 200,000 reads) is obtained from each specimen. The sequence group of the antigen recognition site for the specific antigen can be selected by analyzing an increase or decrease in the number of reads in these time-series data, cluster analysis of similar sequences, or the like using an informatics technique including machine learning.

### 2-1-3. Exhaustive database

A large-scale data set group as schematically shown in Fig. 3 can be constructed by collecting the public database and the independently created database described above so as to cover information on the sequence of the antigen recognition site for the antigen related to any immune reaction (such as infection, cancer immunotherapy, and autoimmune reaction) and additional information. By preparing such a large-scale data set group and by using the antigen receptor repertoire as a biosensor, any immune reaction (such as infection, cancer immunotherapy, and autoimmune reaction) occurring in a living body can be exhaustively captured with a small amount of specimen. An example of using the large-scale data set group includes Form [f].

### 2-2. Sequence identical at level of amino acid sequences or identical at portion excluding two or less amino acid residues (identical sequence)

A database [corresponding to Fig. 1(iii)] of a sequence of an antigen recognition site for a specific antigen and the repertoire data [corresponding to Fig. 1(ii)] are collated with each other to detect, from the repertoire data, a sequence which is identical to the sequence included in the database at a level of amino acid sequences or a sequence which is identical to the sequence included in the database at a portion excluding two or less (preferably one or less) amino acid residues [corresponding to "Sequence identical to database" in Fig. 1]. A sequence which is identical to the sequence included in the database at a level of amino acid sequences or a sequence which is identical to the sequence included in the database at a portion excluding two or less amino acid residues is a sequence corresponding to a completely identical amino acid sequence or an amino acid sequence (similar sequence) including one or two residues (preferably one residue) as mismatches with respect to the sequences in the database.

For detection of an identical sequence, any method capable of comparing amino acid sequences can be used. A specific example of such a method is the Levenshtein distance method. In the Levenshtein distance method, an amino acid sequence having a Levenshtein distance (edit distance, i.e., the minimum number of times of replacement, insertion, and deletion of one character necessary for matching one character string with the other character string) of 0 to 2 (preferably 0 to 1) can be detected. Therefore, among the identical sequences, the Levenshtein distance of the completely identical sequence is 0, and the Levenshtein distance of the similar sequence is 1 to 2 (preferably 1).

### 3. Step C

In step C, the number [corresponding to "Number of identical sequences (clones)" in Fig. 1] and/or frequency [corresponding to "Proportion of identical sequences" in Fig. 1] of sequences specified in the step B in the repertoire data are derived. In the present invention, the step C can optionally be included without depending on the purpose of application of the method of the present invention.

That is, in the step C, it is derived how many sequences (identical sequences) detected as the same or similar sequences as the sequences in the database are included in the repertoire data and/or how frequently the sequences appear. The number and/or frequency can be appropriately derived on the basis of a known method. It can be said that as the number and/or frequency of sequences (identical sequences) specified as sequences identical or similar to the sequences in the database increases in the repertoire data, an immune reaction to a specific antigen is stronger and/or higher in frequency.

In a case where the number and/or frequency of the identical sequences when no immune reaction by a specific antigen occurs is known from common technical knowledge or from the listed information in the database, whether or not the immune reaction occurs can be determined. That is, in this case, the immune reaction can be evaluated even with only one data point. Preferred examples of such a case include Form [b], Form [c], Case e1 of Form [e], and Form [f].

### 4. Step D

In step D, a temporal change in the number and/or frequency, which is obtained in the step C, of sequences identical to the database is confirmed. Examples where the present invention includes the step D include Form [a], Form [b], Form [d], Case e2 and Case e3 of Form [e], and Form [f]. When the method of the present invention is applied to Form [c] or Case e1 of Form [e], the step D is not required.

In the case of evaluating an immune reaction for repertoire data of a subject exposed to a specific antigen (e.g., Form [a], Form [b], Form [f], etc.), it is confirmed whether or not the level of the number and/or frequency obtained in the step C is increased more than the level of the number and/or frequency before the exposure to the antigen. In the case of evaluating an immune reaction for repertoire data of a subject subjected to cancer immunotherapy (e.g., Form [d], Form [f], etc.), it is confirmed whether or not the level of the number and/or frequency obtained in the step C is increased more than the level of the number and/or frequency before the cancer immunotherapy. In the case of evaluating an immune reaction by vaccination for repertoire data of a subject subjected to an immunosuppressive treatment (e.g., Case b4 of Form [b], Form [f], etc.), it is confirmed whether or not the level of the number and/or frequency obtained in the step C is increased or decreased more than the level of the number and/or frequency before the immunosuppressive treatment. In the case of evaluating an immune reaction for repertoire data of a subject suffering from an autoimmune disease (e.g., Case e1 and Case e2 of Form [e], Form [f], etc.), it is confirmed whether or not the level of the number and/or frequency obtained in the step C is increased more than the level of the number and/or frequency in a specimen collected at another timing of the same subject.

As described in the above "1-3. Acquisition time of repertoire data", it has been found that the time of activation of mRNA of an immune cell receptor (i.e., the time of clonal expansion of immune cells in response to a specific antigen in the repertoire) arrives at a predetermined period after stimulation with the specific antigen. Specifically, the time of activation after antigen stimulation for the primary reaction is, for example, 11 to 20 days or 11 to 18 days, preferably 12 to 16 days, and more preferably 13 to 15 days, and the time of activation after antigen stimulation for the secondary and subsequent reactions is, for example, 3 to 10 days or 4 to 10 days, preferably 5 to 9 days, and more preferably 6 to 8 days. Therefore, it is possible to determine whether the stimulation by the specific antigen has caused the primary reaction or the secondary and subsequent reactions by confirming whether the time when an increase in the number and/or frequency of sequences identical to the database is observed corresponds to any one of the time of activation after the antigen stimulation for the primary reaction and the time of activation after the antigen stimulation for the secondary and subsequent reactions. Specifically, when an increase in the number and/or frequency of sequences identical to the database is observed 11 to 20 days or 11 to 18 days (preferably 12 to 16 days, more preferably 13 to 15 days) after stimulation with a specific antigen, an immune reaction by the stimulation can be determined to be the primary reaction. When an increase in the number and/or frequency of sequences identical to the database is observed 3 to 10 days (preferably 5 to 9 days, more preferably 6 to 8 days) after stimulation with a specific antigen, the immune reaction by the stimulation can be determined to be the secondary and subsequent reactions.

### 5. Step E, step E', step F, step G

When the method of the present invention includes the above steps A to D, the method can further include other steps as long as the effects of the present invention are not impaired. Other steps can be determined according to the application purpose of the method of the present invention.

### 5-1. Step E and step E'

The method of the present invention can further include step E and/or step E' when the immune reaction is evaluated for repertoire data of a subject vaccinated with a nucleic acid vaccine. Examples of a case where the step E is included include Case a1 to Case a3 of Form [a] and Form [f]. Examples of a case where the step E' is included include Case a4 of Form [a] and Form [f].

In the step E, the scientific validity of the nucleic acid vaccine is evaluated by confirming the information listed in the database with respect to the sequence in which an increase is confirmed as a temporal change in the number and/or frequency of the sequence identical to the database. The information listed in the database is selected from the group consisting of [1] information on the presence or absence of neutralizing activity against an antigen, [2] information on a species or strain of an antigen, and [3] information on an epitope. The evaluating of the scientific validity is selected from the group consisting of [1] evaluating that the nucleic acid vaccine has clinical effectiveness of producing an antibody having neutralizing activity when it is confirmed that the neutralizing activity exists, [2] evaluating that the nucleic acid vaccine is compatible with a target antigen when it is confirmed that the species or strain is the same as a species or strain of the target antigen in design of the nucleic acid vaccine, and [3] evaluating that the nucleic acid vaccine is compatible with a target epitope when it is confirmed that the epitope is the same as the target epitope in design of the nucleic acid vaccine. The case of the above [1] corresponds to Case a1 of Form [a], the case of the above [2] corresponds to Case a2 of Form [a], and the case of the above [3] corresponds to Case a3 of Form [a].

By the step E, as for a nucleic acid vaccine containing a design drawing (genetic information) of a specific antigen as an active ingredient, it is possible to confirm scientific validity that an antibody having a neutralizing activity can be produced in vivo, an antibody capable of recognizing a target antigen (species or strain) as designed can be produced, or an antibody capable of recognizing a target epitope as designed can be produced.

In the step E', the nucleic acid vaccine is evaluated to have an ability to produce an antibody having an antigen recognition site completely identical to that of an antibody by adaptive immunity by infection, as the scientific validity of the nucleic acid vaccine, when the sequence in which an increase is confirmed as the temporal change in the number and/or frequency of sequences identical to the database is identical (i.e., completely identical) to the sequence included in the database at a level of amino acid sequences. According to the present invention, it is possible to detect a large number of sequences in which the sequences of the antigen recognition site in the immune cell receptor are completely identical to each other. When the amino acid sequence of the sequence of the antigen recognition site is, for example, a sequence consisting of 10 amino acids, since there are 20 types of amino acids, in view of the fact that theoretically 20 to the power of 10 types of sequences are required to find a completely identical sequence, it is recognized as a surprising efficiency that a large number of completely identical sequences can be detected according to the present invention.

By the step E', as shown in Fig. 4, as for the nucleic acid vaccine vaccinated into a subject which is a healthy individual, it is possible to confirm the scientific validity that an antibody that is completely identical to an antibody acquired by a patient by infection with an antigen such as a virus (having a sequence included in the database) can be produced in the body of the subject who is a healthy individual by vaccination.

### 5-2. Step F

The method of the present invention can further include step F when the immune reaction is evaluated for repertoire data of a subject vaccinated with a vaccine (i.e., a specific antigen), the subject retaining another antibody (i.e., an antibody different from an antibody produced by vaccination with a vaccine as a specific antigen) at a high level at the time of specimen collection. Examples of a case where the step F is included include Case b1 to Case b3 of Form [b] and Form [f].

The subject in this case can be selected from the subjects (S1) to (S3) mentioned in the above "1-2-1-3. Subject retaining another antibody at high level", and specifically includes a subject vaccinated with a vaccine as a specific antigen, the subject being administered with an antibody drug, vaccinated with another vaccine, and/or infected.

In the step F, it is determined that the vaccine works when the number and/or frequency of sequences identical to the database increases over time. Here, as described in the above "1-2-1-3. Subject retaining another antibody at high level", due to administration of an antibody drug, vaccination with another vaccine, or infection, another antibody (specifically, an antibody related to an antibody drug, an antibody produced in an immune reaction to another vaccine, or an antibody produced in an immune reaction to a pathogen) is present at a high level in the bodies of the subject (S1) to the subject (S3). Since the half-life of the antibody protein is long, these other antibodies are retained at a high level in the bodies of the subject (S1) to the subject (S3). In this case, even when an antibody is generated by an immune reaction to a vaccine as a specific antigen separately in parallel, such an antibody is masked by a large amount of other coexisting antibodies, and thus cannot be confirmed by conventional repertoire analysis or an immunological measurement method such as ELISA. Since the method of the present invention can selectively specify an immune reaction to a specific antigen from the repertoire data without being affected by the amount of antibody protein in the body, even in a subject retaining another antibody at a high level, the immune reaction can be selectively specified as long as an immune reaction to a vaccine as a specific antigen occurs.

### 5-3. Step G

The method of the present invention can further include step G when the immune reaction is evaluated for repertoire data of a subject subjected to an immunosuppressive treatment such as hematopoietic stem cell transplantation or B cell depletion therapy. Examples of a case where the step G is included include Case b4 of Form [b] and Form [f].

In the step F, it is possible to further include step G of determining the presence or absence of restoration of immune function on the basis of whether or not the temporal change in the number and/or frequency of sequences identical to the database has increased. For example, when an increase in the number and/or frequency of sequences identical to the database is observed in repertoire data of subjects subjected to an immunosuppressive treatment and then vaccinated with a vaccine as a specific antigen, it is possible to confirm restoration of immune function that has been reduced by the immunosuppressive treatment.

### 6. Step H

In a case where the repertoire data prepared in the step A is acquired from a specimen of a subject vaccinated with a vaccine (existing vaccine) against a previously-encountered viral strain ST1, and the database used for collation in the step B includes a group of sequences of antigen recognition sites in immune cell receptors for a viral strain ST2 (e.g., a mutant strain of the viral strain ST1, etc.) whose effectiveness by the vaccine (existing vaccine) is unknown, the present invention can further include step H of evaluating that the vaccine (existing vaccine) is also effective against the viral strain ST2 when the sequence detected in the step B is present. In this case, it is possible to determine that the existing vaccine is effectively used for the newly appeared viral strain. Conversely, when no sequence was detected by the step B, it is possible to determine that a new vaccine needs to be developed.

### Examples

Hereinafter, the present invention will be more specifically described by means of Examples; however, the present invention is not limited thereto.

### [Test Example 1]

### [Materials and methods]

### Patient

To evaluate the immune reaction after SARS-CoV-2 infection, peripheral blood samples were collected in time series from three COVID-19 patients at Kobe University Hospital from August 2020 to December 2020. All the patients were classified as mild and no immunosuppressive agents were administered. COVID-19 was confirmed by quantitative reverse transcription-polymerase chain reaction assay (rtPCR) on SARS-CoV-2.

To evaluate the immune reaction after mRNA SARS-CoV-2 vaccination, healthy volunteers were enrolled between May 2021 and December 2022. The SARS-CoV-2 mRNA vaccine used for all participants is monovalent BNT162b2 [B.1.1.529], bivalent BNT162b2 [WT/OMI BA.4-5], or monovalent mRNA-1273. These participants were confirmed to be uninfected with SARS-CoV-2 by measuring anti-SARS-CoV-2 nucleocapsid protein IgG antibody before vaccination. Blood samples were taken in time series before and after vaccination.

In order to examine the anti-SARS-CoV-2 spike antibody titer in a serum sample after administration of the neutralizing antibody drug tixagevimab/cilgavimab for COVID-19 prevention, 19 patients with hematologic malignancies (17 patients in Fig. 1, and 2 hematopoietic stem cell transplantation patients for whom BCR repertoire has been analyzed in Figs. 4A and 4B) for which tixagevimab/cilgavimab was indicated were enrolled. Blood samples were collected in time series before and after tixagevimab/cilgavimab administration.

### Sample collection and treatment

Peripheral blood samples were collected using heparin-containing tubes. Peripheral blood mononuclear cells (PBMCs) were separated from blood by density gradient centrifugation using Ficoll-Paque Plus (GE Healthcare, Little Chalfont, UK) and SepMate-50 tubes (STEMCELL Technologies, Vancouver, Canada) according to the manufacturer's protocol (STEMCELL Technologies). PBMC samples were stored at -80°C until analysis using CELLBANKER (ZENOGEN PHARMA, Fukushima, Japan). Total RNA was extracted with TRIzol LS (Thermo Fisher Scientific, Waltham, MA, USA) and purified using RNeasy Mini Kit (Qiagen, Hilden, Germany) according to the manufacturer's protocol. RNA amount and purity were measured using Agilent 2200 TapeStation (Agilent Technologies, Santa Clara, CA, USA). Blood samples were centrifuged at 1000×g for 10 minutes at room temperature and immediately transferred to a freezer kept at -80°C, thereby obtaining serum samples.

### SARS-CoV-2 specific immunoglobulin antibody assay

Three fully automated commercial immunoassays were used. Abbott SARS-CoV-2 IgG II Quant (Abbott Laboratories, Sligo, Ireland) is a chemiluminescent microparticle immunoassay (CMIA) designed for the quantitative determination of IgG antibodies against the receptor-binding domain (RBD) of the S1 subunit of SARS-CoV-2 spike proteins. The test was performed on the Abbott Architect i2000SR system (Abbott Laboratories). Roche Elecsys anti-SARS-CoV-2 S (Roche Diagnostics, Basel, Switzerland) is an electrochemiluminescent immunoassay (ECLIA) to quantitatively determine total Ig antibodies against the RBD of SARS-CoV-2 spike proteins. The test was performed on a Roche Cobas e601 system (manufactured by Roche Diagnostics K.K.). Atellica IM SARS CoV-2 IgG (Siemens Healthcare Diagnostics, Erlangen, Germany) is a chemiluminescent immunoassay (CLIA) designed to quantitatively determine IgG antibodies against the RBD of the S1 subunit of SARS-CoV-2 spike proteins. The test was performed on an Atellica IM automated analyzer (Siemens Healthcare Diagnostics).

The antibody titer against nucleocapsid protein was measured by QuaResearch COVID-19 Human IgM IgG ELISA Kit (nucleocapsid protein) (Cellspect, Inc., RCOEL961-N, Iwate, Japan). This kit detects an antibody titer on the basis of an indirect ELISA method, and is attached with a kit in which different antigenic proteins are immobilized. In the plate of the ELISA kit (nucleocapsid protein), recombinant nucleocapsid protein (full length) of SARS-CoV-2 expressed in E. coli is immobilized. Nucleocapsid protein in the serum sample was measured according to the manufacturer's measurement protocol.

### Flow cytometric analysis

PBMCs were stained for 20 minutes at 4°C using the following anti-human antibodies: CD3 APC, CD4 BV510 and CD8 BV711 (all BD Biosciences, San Diego, CA, USA) for T cell lines and CD19 BV510, CD27 BV421, IgD BV711 and CD38 BV510 (all Biolegend, San Diego, CA, USA) for B cell lines. Isotype matched antibodies were used as controls. Flow cytometric analysis was performed using BD FACSAria III device (BD Biosciences). CD3+CD4+ cells were defined as helper T cells. CD19+CD27+IgD- was defined as class-switched B cell. CD19+CD27+CD38+ was defined as plasma blast.

### B cell receptor repertoire analysis

BCR repertoire analysis was performed using an unbiased next-generation sequencer developed by Repertoire Genesis Inc. In short, cDNA was synthesized from total RNA using polyT18 primer (BSL-18E) and Superscript III reverse transcriptase (Invitrogen, CA, USA). After double-stranded (ds)-cDNA was synthesized, P10EA/P20EA dsDNA adaptors were ligated and cleaved with NotI restriction enzymes. Nested-PCR was performed with IgG constant region specific primers (CG1 and CG2) and P20EA using KAPA HiFi DNA Polymerase (Kapa Biosystems, Woburn, MA, USA). Amplicon libraries were prepared by amplifying the second PCR product using P22EA-ST1 and CG-ST1-R. Index (barcode) sequences were added by amplification using Nextera XT Index Kit v2 Set A (Illumina, San Diego, CA, USA). Sequencing was performed using an Illumina MiSeq paired-end platform (2×300 bp). BCR sequences were assigned on the basis of identity to a reference sequence in the international ImMunoGeneTics information system^{(R)} (IMGT) database (http://www.imgt.org) using a repertoire analysis software independently developed by Repertoire Genesis Inc. (Osaka, Japan).

### Calculation of index and network analysis

Data analysis and graphing used a package implemented in R software (version 4.0.2). The Levenshtein distance (edit distance) was calculated using the stringdist 0.9.10 package. The network analysis was performed using igraph 1.2.6 (https://igraph.org/r/) implemented in R. The 1,000th most frequent BCR clone types (nodes) were connected by an edge defined by one amino acid sequence or less of CDR3 or a Levenshtein distance of one amino acid sequence or less. The network was created using the force-directed layout algorithm by Fruchterman and Reingold. Graphics were drawn using ggplot2 version 3.3.2. Generation probability (pGen) values for human IGH sequences were calculated using the OLGA package (Sethna Z, Elhanati Y, Callan CG, Walczak AM, Mora T. OLGA: fast computation of generation probabilities of B- and T-cell receptor amino acid sequences and motifs. Bioinformatics. 2019; 35(17):2974-2981.).

### Search of database and sequence specific for COVID-19

Antibody sequences specific for COVID-19 were downloaded from CoV-AbDab (http://opig.stats.ox.ac.uk/webapps/covabdab/). Data updated on December 20, 2022 with 12,004 entries were used as reference. Unpaired antibody sequences reported between 2011 and 2021 were downloaded from The Observed Antibody Space database (OAS, http://opig.stats.ox.ac.uk/webapps/oas/) on the basis of the "heavy chain" and "IGHG" attributes. A total of 260,856,092 sequences were used for validation of the method. A total of 1,259,140 IgG antibody sequences from 12 healthy volunteers before the COVID-19 pandemic have already been reported (Kitaura K, Yamashita H, Ayabe H, Shini T, Matsutani T, Suzuki R. Different Somatic Hypermutation Levels among Antibody Subclasses Disclosed by a New Next-Generation Sequencing-Based Antibody Repertoire Analysis. Front Immunol. 2017; 8:389.). Sequences with 0 to 2 amino acid sequence differences (Levenshtein distance, "CDR3AA Distance") in CDR3 (identical sequences) with identical V and J gene names to the query sequences were acquired from the database. Of CoV-AbDab, the sequence having the "Binds to" or "Neutralizing Vs" attribute of SARS-CoV2 was a SARS-CoV-2 specific sequence regardless of the variant. A sequence having the "Neutralizing Vs" attribute of SARS-CoV2 was classified as a neutralizing antibody (Neut+), and a sequence in which SARS-CoV-2 is not included in the "Neutralizing Vs" attribute but SARS-CoV-2 is included in the "Not Neutralizing Vs" attribute was classified as a non-neutralizing antibody (Neut-).

### [Result]

### Influence of tixagevimab/cilgavimab administration on anti-SARS-CoV-2 spike IgG antibody measurement by ELISA method

In order to examine the influence of tixagevimab/cilgavimab on anti-SARS-CoV-2 spike antibody measurement, antibody measurement was performed in time series before and after administration with three representative automated quantitative anti-SARS-CoV-2 spike immunoassays using different ELISA methods (Abbott Laboratories, Roche Diagnostics, Siemens Healthcare Diagnostics). 17 patients with hematologic malignancies (B cell depletion therapy n = 8, hematopoietic stem cell transplantation n = 6, chemotherapy n = 3) for which tixagevimab/cilgavimab was indicated were enrolled. As expected, the antibody titers after administration were extremely high in all cases, and high values were maintained over a long period of time (Fig. 1).

### Analysis of OAS public database using CoV-AbDab

Previously reported repertoire data before and after the COVID-19 pandemic were used to validate the usefulness of CoV-AbDab. From the obtained 12,004 CoV-AbDab data, SARS-CoV-2 specific sequences were retrieved from public databases and data of the cohort of patients described above using a look-up table with 8,977 identical V and J gene names and CDR3 amino acids of human sequences (Fig. 2A). First, a total of 260,856,092 sequences published between 2011 and 2021 from the OAS public database were analyzed using CoV-AbDab. The V and J gene names and complete sequence matching of CDR3 amino acid sequences were hardly detected in the data before pandemic with only 132 sequences (0.00019%) out of 67,856,692 sequences before 2019; on the other hand, more sequences (0.022%, 1,817 sequences out of 8,441,259 sequences) were detected after 2020 (Table 1, frequency of occurrence (%) of SARS-CoV-2 specific sequences in the OAS database, numbers in parentheses indicate data of samples whose Disease attributes are classified as SARS-CoV-2). In order to detect sequences similar to the SARS-CoV-2 specific sequences, antibody sequences with Levenshtein (LV) distance with amino acid difference of CDR3 of 1 or 2 were searched. 4,641 (0.0068%) similar sequences with an LV distance of 1 were detected before 2019 and 12,251 (0.15%) similar sequences with an LV distance of 1 were detected after 2020. 59,356 (0.087%) similar sequences with an LV distance of 2 were detected before 2019 and 27,484 (0.33%) similar sequences with an LV distance of 2 were detected after 2020. When the detection rate after 2020 with respect to before 2019 is taken as an S/N ratio, the best S/N ratio was 110.6 for the completely identical sequence (LVO), 21.2 for LV1, and 3.7 for LV2. The sensitivity and the S/N ratio change depending on the distance of the CDR3 sequence. These results showed that, in order to detect the SARS-CoV-2 specific antibody sequence from the antibody repertoire data, a computational approach using the CoV-AbDab reference sequence is effective.

**[Table 1]**

| | | | %Frequency | | |
|---|---|---|---|---|---|
| | | | CDR3 levenshtein distance | | |
| Year | Entry | Unique sequence | 0 | 1 | 2 |
| 2011 | 3 | 37856 | 0.0000 | 0.0000 | 0.0687 |
| 2012 | 3 | 26242 | 0.0000 | 0.0000 | 0.0152 |
| 2013 | 44 | 502900 | 0.0000 | 0.0074 | 0.0871 |
| 2014 | 79 | 725327 | 0.0000 | 0.0043 | 0.0545 |
| 2015 | 299 | 3847328 | 0.0001 | 0.0058 | 0.0705 |
| 2016 | 437 | 6408509 | 0.0002 | 0.0045 | 0.0542 |
| 2017 | 132 | 1666985 | 0.0000 | 0.0022 | 0.0377 |
| 2018 | 97 | 993132 | 0.0001 | 0.0072 | 0.0769 |
| 2019 | 579 | 53648413 | 0.0002 | 0.0074 | 0.0949 |
| 2020 | 326 (155) | 6234720 (3857508) | 0.0196 (0.0314) | 0.1126 (0.1793) | 0.2812 (0.4286) |
| 2021 | 119 (99) | 2206539 (1968418) | 0.0270 (0.0169) | 0.2370 (0.1011) | 0.4510 (0.1944) |

Next, the data of healthy individuals before the COVID-19 pandemic was also uniquely analyzed. A total of 1,259,140 in-frame sequences of 12 healthy individuals (age: 37 ± 11.7 [average ± SD], gender: 11 : 1 [male : female]) were analyzed using CoV-AbDab. The percentage frequencies of these sequences in each sample were 0.000075 ± 0.00026% (LV = 0), 0.00043 ± 0.00076% (LV = 1), and 0.0065 ± 0.0032% (LV = 2). From these results, it is considered that SARS-CoV-2 specific sequences are hardly detected in uninfected healthy individuals, and even if detected, the frequency is considerably low (Table 2, Frequency (%) of SARS-CoV-2 specific sequences in healthy individuals prior to the pandemic).

**[Table 2]**

| | | | %Frequency | | |
|---|---|---|---|---|---|
| | | | CDR3 Levenshtein distance | | |
| Donor | In-frame total | In-frame unique | 0 | 1 | 2 |
| HI01 | 145453 | 21211 | 0 | 0 | 0.0069 |
| HI02 | 93493 | 17939 | 0 | 0 | 0.0075 |
| HI03 | 110929 | 23798 | 0 | 0 | 0.013 |
| HI04 | 136325 | 13267 | 0 | 0.00073 | 0.0059 |
| HI05 | 110450 | 11253 | 0.00091 | 0 | 0.0018 |
| HI06 | 99787 | 18114 | 0 | 0.0020 | 0.0060 |
| HI07 | 67445 | 10554 | 0 | 0.0015 | 0.0089 |
| HI08 | 39117 | 9130 | 0 | 0 | 0.0077 |
| H109 | 129626 | 13390 | 0 | 0 | 0.0015 |
| HI10 | 122188 | 15617 | 0 | 0 | 0.0049 |
| HI11 | 93366 | 14410 | 0 | 0 | 0.0096 |
| HI12 | 110961 | 23306 | 0 | 0.00090 | 0.0045 |

### Analysis of BCR repertoire data of COVID-19 patient using CoV-AbDab

It was attempted whether the immune response after SARS-CoV-2 infection could be tested by BCR repertoire analysis using CoV-AbDab. Analysis of BCR repertoire was performed on 17 blood samples collected from three patients with primary COVID-19. A total of 4,768,753 sequences were obtained, of which 2,858,278 in-frame sequences with amino acid assignments of V and J genes and CDR3 were used. Next, it was analyzed whether there was a sequence identical to the CDR3 sequence of CoV-AbDab among the CDR3 sequences (Fig. 2B). As expected, some sequences identical to the CDR3 sequence of CoV-AbDab were detected from patient samples. Although an identical sequence was not detected immediately after onset, the sequence gradually increased after onset, and reached a peak around two weeks (Fig. 2B).

The network formed by the SARS-CoV-2 specific BCR clone type was also analyzed. Similar SARS-CoV-2 specific sequences bound each other and clustered. BCR clusters rapidly formed and grew, and reached a maximum size in about 2 weeks after onset of symptoms. Thereafter, the clusters disappeared rapidly (Fig. 2B).

### Analysis of BCR repertoire data of healthy volunteers vaccinated with mRNA SARS-CoV-2 vaccine using CoV-AbDab

To verify whether BCR repertoire analysis using CoV-AbDab could detect a post-vaccination immune response, BCR repertoire was analyzed on blood samples of healthy volunteers who were vaccinated the first and second time with mRNA SARS-CoV-2 vaccine (monovalent BNT162b2 [B.1.1.529], Pfizer).

First, the dominant clonal type was focused by BCR repertoire data without using CoV-AbDab, but no significant change was detected before and after vaccination (Fig. 7).

Next, in BCR repertoire analysis using CoV-AbDab, the number of matched sequences and the percentage frequency were analyzed. As a result, it was found that the number of matched sequences increased and immediately decreased 2 weeks after the first vaccination (Fig. 3A). On the other hand, after the second vaccination, the number of identical sequences increased more rapidly (1 week) (Fig. 3A). Similar results were also observed for booster shots of other mRNA vaccinations (bivalent BNT162b2 [WT/OMI BA.4-5, Pfizer] and monovalent mRNA-1273 [Moderna]) (Figs. 3B and 3C). Thus, BCR repertoire analysis using CoV-AbDab detected a post-vaccination immune response in healthy volunteers after vaccination as well as in COVID-19 patients.

### Reactivity to mRNA SARS-CoV-2 vaccination in patients after administration of tixagevimab/cilgavimab

In two patients with hematologic malignancies who received allogeneic hematopoietic stem cell transplantation, the reactivity to mRNA SARS-CoV-2 vaccination after administration of tixagevimab/cilgavimab (T/C) was evaluated by BCR repertoire analysis by CoV-AbDab.

Case 1 (T/C patient 1): A 63 year old female who developed acute myeloid leukemia with myelodysplasia-associated changes received cord blood transplantation (CBT). Tixagevimab/cilgavimab was administered on Day 338 after CBT and the fourth vaccination (second vaccination after CBT) of mRNA vaccine (bivalent BNT162b2) was performed on Day 348 after CBT.

Case 2 (T/C patient 2): A 40 year old male who developed B-cell acute lymphoblastic leukemia received unrelated bone marrow transplantation (u-BMT). Tixagevimab/cilgavimab was administered on Day 212 after u-BMT and the first mRNA vaccine (monovalent BNT162b2) was administered on Day 218 after u-BMT.

The vaccination reaction after the hematopoietic stem cell transplantation depends on reconstitution of T cells and B cells. At the time of vaccination, flow cytometry analysis was performed to evaluate reconstitution of T cells and B cells. The presence of helper T cells (CD3+CD4+), class-switched B cells (CD19+CD27+IgD-), and plasma blasts (CD19+CD27+CD38+) was confirmed (Figs. 4A and 4B). BCR repertoire analysis using CoV-AbDab clearly detected a reaction to mRNA SARS-CoV-2 vaccination even after the administration of tixagevimab/cilgavimab (Figs. 4A, 4B, and 8).

### Characteristics of SARS-CoV-2 specific sequences in infected individuals and vaccinated individuals

Finally, the SARS-CoV-2 specific sequence was characterized. First, the neutralizing ability or the non-neutralizing ability was determined from the attributes of the reference sequence (Fig. 5A). The neutralizing ability of the induced antibodies differed between groups, with more neutralizing antibodies induced in vaccinated individuals and more non-neutralizing antibodies induced in a few infected individuals. The frequency of individual SARS-CoV-2 specific sequences included in each sample was examined, and the frequency ratio of the completely identical sequence was significantly higher than that of the sequence in which 1 to 2 amino acids are not identical (average ± SD: 0.029 ± 0.088 at LV0, 0.0079 ± 0.034 at LV1, 0.0074 ± 0.041 at LV2, Kruskal-Wallis test and Dunn-Bonferroni post hoc test, LV0 vs. LV1: p < 0.0001, LVO vs. LV2: p < 0.0001, LV2 vs. LV3: not significant) (Fig. 5B). The use rate of the CDR3 length of the identical antibody sequences and the combination of V and J was examined (Figs. 5C and 5D). The most completely identical sequence was IGHV4-59/IGHJ4, followed by IGHV3-33/IGHJ4. The sequence including one amino acid mismatch had IGHV1-8/IGHJ4, IGHV3-30-3/IGHJ4, or IGHJ6, and IGHV3-9/IGHJ4, or IGHV3-33/IGHJ4 with a high frequency. These SARS-CoV-2 specific sequences were detected in multiple individuals and showed public antibody properties (Tables 3A and 3B, SARS-CoV-2 specific sequences of infected individuals and vaccinated individuals (completely identical)). Only 86 sequence species were detected as SARS-CoV-2 specific sequences. Most of the completely identical sequences were found to be short CDR3 of 8 amino acids and IGHV4-59/IGHJ4 sequences (Fig. 6A). Among the known SARS-CoV-2 specific sequences, those having a high generation probability (pGen) were often found in infected individuals and vaccinated individuals (Fig. 6B). These patterns were similar to the search results in the OAS database, demonstrating the validity of the method of the present example (Figs. 9 to 12).

**[Table 3A]**

| IGHV | IGHJ | CDR3 | CDR3 Length | Read | Number of individuals detected |
|---|---|---|---|---|---|
| IGHV4-59 | IGHJ4 | CARGFDYW | 8 | 3264 | 5 |
| IGHV3-33 | IGHJ4 | CAREGIVGATTGFDYW | 16 | 643 | 3 |
| IGHV4-59 | IGHJ2 | CARGFDLW | 8 | 499 | 3 |
| IGHV4-59 | IGHJ5 | CARGFDPW | 8 | 262 | 3 |
| IGHV3-9 | IGHJ4 | CAKDIGYSSGLFDYW | 15 | 218 | 2 |
| IGHV3-53 | IGHJ6 | CARDLVDYGMDVW | 13 | 191 | 1 |
| IGHV4-59 | IGHJ4 | CARGFDFW | 8 | 165 | 2 |
| IGHV3-66 | IGHJ4 | CARDYGDFYFDYW | 13 | 150 | 3 |
| IGHV3-33 | IGHJ4 | CARGSGSGDYW | 11 | 142 | 2 |
| IGHV3-53 | IGHJ4 | CARDYGDFYFDYW | 13 | 130 | 3 |
| IGHV1-58 | IGHJ3 | CAAPYCSGGSCSDAFDIW | 18 | 112 | 1 |
| IGHV3-20 | IGHJ4 | CARGSGSLDYW | 11 | 104 | 1 |
| IGHV3-53 | IGHJ6 | CARDLYYYGMDVW | 13 | 69 | 3 |
| IGHV3-20 | IGHJ4 | CARGSGSSDYW | 11 | 66 | 2 |
| IGHV3-15 | IGHJ4 | CTTHSTPDYW | 10 | 64 | 2 |
| IGHV3-15 | IGHJ4 | CTTHSSPDYW | 10 | 57 | 1 |
| IGHV3-30-3 | IGHJ6 | CARARGGNYYYGMDVW | 16 | 54 | 3 |
| IGHV3-66 | IGHJ3 | CARETYAFDIW | 11 | 50 | 1 |
| IGHV4-59 | IGHJ4 | CARGFDSW | 8 | 48 | 2 |
| IGHV3-53 | IGHJ6 | CARDLVVYGMDVW | 13 | 43 | 2 |
| IGHV3-53 | IGHJ6 | CARDLGPRYGMDVW | 13 | 41 | 1 |
| IGHV1-2 | IGHJ6 | CARYSNYYYYYGMDVW | 16 | 35 | 2 |
| IGHV4-59 | IGHJ5 | CARGFDSW | 8 | 32 | 1 |
| IGHV4-59 | IGHJ5 | CARGFDYW | 8 | 30 | 2 |
| IGHV3-20 | IGHJ4 | CARGSGGLDYW | 11 | 28 | 1 |
| IGHV3-33 | IGHJ4 | CAREGQVGATTGLDYW | 16 | 24 | 1 |
| IGHV3-7 | IGHJ4 | CARLGGSSWHFDYW | 14 | 23 | 2 |
| IGHV4-59 | IGHJ3 | CARGFDIW | 8 | 22 | 2 |
| IGHV1-18 | IGHJ4 | CALVGATDYW | 10 | 20 | 1 |
| IGHV3-33 | IGHJ4 | CAREGAVGATTGFDYW | 16 | 19 | 1 |
| IGHV3-66 | IGHJ4 | CARDYGDYYFDYW | 13 | 18 | 2 |
| IGHV3-53 | IGHJ6 | CARDLIDYGMDVW | 13 | 17 | 1 |
| IGHV4-59 | IGHJ4 | CVRGFDYW | 8 | 16 | 3 |
| IGHV3-33 | IGHJ4 | CAREGQVGATTGFDYW | 16 | 14 | 1 |
| IGHV4-34 | IGHJ6 | CARVGGYYYYYMDVW | 15 | 13 | 1 |
| IGHV3-20 | IGHJ4 | CARGTGSLDYW | 11 | 12 | 1 |
| IGHV3-30 | IGHJ6 | CARARGGSYYYGMDVW | 16 | 12 | 2 |
| IGHV3-33 | IGHJ4 | CARGSGSSDYW | 11 | 12 | 1 |
| IGHV1-18 | IGHJ5 | CARDGELLGWFDPW | 14 | 11 | 1 |
| IGHV3-30-3 | IGHJ4 | CARDGGGYFDYW | 12 | 11 | 1 |
| IGHV3-9 | IGHJ6 | CAKDGGVNWGNDYGMDVW | 18 | 11 | 1 |
| IGHV3-30-3 | IGHJ4 | CARGLGGNYYYFDYW | 15 | 10 | 1 |
| IGHV3-66 | IGHJ4 | CARGYGDYYFDYW | 13 | 10 | 1 |

**[Table 3B]**

| IGHV | IGHJ | CDR3 | CDR3 Length | Read | Number of individuals detected |
|---|---|---|---|---|---|
| IGHV1-58 | IGHJ3 | CAAPYCSGGSCYDGFDIW | 18 | 9 | 1 |
| IGHV1-58 | IGHJ3 | CAAPYCSGGTCNDGFDIW | 18 | 9 | 1 |
| IGHV3-13 | IGHJ4 | CARGOSSGYYYYFDYW | 16 | 9 | 1 |
| IGHV3-30-3 | IGHJ4 | CARGRGNYLTYFDYW | 15 | 9 | 1 |
| IGHV3-66 | IGHJ4 | CARDLGGYFDYW | 12 | 9 | 1 |
| IGHV3-30 | IGHJ6 | CARARGGNYYYGMDVW | 16 | 8 | 3 |
| IGHV3-53 | IGHJ4 | CARGYGDYYFDYW | 13 | 8 | 1 |
| IGHV3-33 | IGHJ4 | CAREGQVGATTGIDYW | 16 | 6 | 1 |
| IGHV3-53 | IGHJ4 | CARGEGWDLPFDYW | 14 | 6 | 1 |
| IGHV3-53 | IGHJ6 | CARGGGHYYGMDVW | 14 | 6 | 1 |
| IGHV3-7 | IGHJ4 | CARVGSSSWYFDYW | 14 | 6 | 3 |
| IGHV1-46 | IGHJ6 | CARGGSHGMDVW | 12 | 5 | 1 |
| IGHV3-30-3 | IGHJ4 | CARGLGGNYYYFDFW | 15 | 5 | 1 |
| IGHV3-30-3 | IGHJ6 | CARARGGGYYYGMDVW | 16 | 5 | 1 |
| IGHV3-66 | IGHJ4 | CARSYGDYYFDYW | 13 | 5 | 1 |
| IGHV3-66 | IGHJ6 | CARDLVDYGMDVW | 13 | 5 | 1 |
| IGHV1-69 | IGHJ2 | CARERGYSGYGASWYFDLW | 19 | 3 | 1 |
| IGHV3-20 | IGHJ6 | CARGGGGMDVW | 11 | 3 | 1 |
| IGHV3-30-3 | IGHJ3 | CARNYYDSSDAFDIW | 15 | 3 | 1 |
| IGHV3-53 | IGHJ3 | CARVVSDAFDIW | 12 | 3 | 2 |
| IGHV3-20 | IGHJ4 | CARGVGALDYW | 11 | 2 | 1 |
| IGHV3-30-3 | IGHJ3 | CARARGGSYSDAFDIW | 16 | 2 | 1 |
| IGHV3-30-3 | IGHJ3 | CARPRGGNYFDAFDtW | 16 | 2 | 1 |
| IGHV3-30-3 | IGHJ6 | CARATRGSYYYGMDVW | 16 | 2 | 1 |
| IGHV3-30-3 | IGHJ6 | CARAYGGNYYYGMDVW | 16 | 2 | 1 |
| IGHV3-53 | IGHJ4 | CARDYGDYYFDYW | 13 | 2 | 1 |
| IGHV3-53 | IGHJ6 | CARESYGMDVW | 11 | 2 | 1 |
| IGHV4-59 | IGHJ5 | CARGFESW | 8 | 2 | 2 |
| IGHV1-58 | IGHJ3 | CAAPNCSGGTCYDGFDIW | 18 | 1 | 1 |
| IGHV3-30 | IGHJ4 | CAREDYYDSSGSFDYW | 16 | 1 | 1 |
| IGHV3-30 | IGHJ4 | CARGLSGNYYYFDYW | 15 | 1 | 1 |
| IGHV3-30 | IGHJ5 | CARDLGSGWYPW | 12 | 1 | 1 |
| IGHV3-30 | IGHJ6 | CAKARGGSYYYGMDVW | 16 | 1 | 1 |
| IGHV3-30 | IGHJ6 | CARSRGGGYYYGMDVW | 16 | 1 | 1 |
| IGHV3-30-3 | IGHJ4 | CAREDYYDSSGSLDYW | 16 | 1 | 1 |
| IGHV3-30-3 | IGHJ4 | CARPHSGSYRGRFDYW | 16 | 1 | 1 |
| IGHV3-30-3 | IGHJ4 | CARSHSGSYFSYFDYW | 16 | 1 | 1 |
| IGHV3-53 | IGHJ3 | CARSYDILTGYRDAFDIW | 18 | 1 | 1 |
| IGHV3-53 | IGHJ6 | CARDLGPSGMDVW | 13 | 1 | 1 |
| IGHV3-53 | IGHJ6 | CARDLVSYGMDVW | 13 | 1 | 1 |
| IGHV3-66 | IGHJ4 | CARDYGDFYFDFW | 13 | 1 | 1 |
| IGHV3-66 | IGHJ6 | CARDLVAYGMDVW | 13 | 1 | 1 |
| IGHV4-59 | IGHJ4 | CTRGFDYW | 8 | 1 | 1 |

### [Regarding effects of present invention]

This time, using "BCR repertoire data" and "database of BCR sequences binding to target antigens", mRNA SARS-CoV-2 vaccine reactions were evaluated at the level of mRNA expression, not protein expression. In fact, this method for quantification of antigen-specific antibody sequences clearly showed that there was also a response to mRNA SARS-CoV-2 vaccination after the administration of tixagevimab/cilgavimab (Figs. 4A and 4B). Circulating variants change over time in individual regions. In January 2023, the FDA revised its emergency use authorization for tixagevimab/cilgavimab because tixagevimab/cilgavimab was confirmed to be ineffective against more than 90% of the variants currently circulating in the United States. Currently, a variety of anti-SARS-CoV-2 monoclonal antibodies are being developed against new variants, including an omicron strain, other than tixagevimab/cilgavimab. The type of antibody preparation to be applied in each country varies depending on viral mutation, but it can be inferred that the method of the present example is applicable after administration of any type of anti-SARS-CoV-2 antibody.

The establishment of this method for detecting vaccine reactions after antibody administration is of great clinical significance. Since antigens developed in mRNA vaccines may be occupied by a large amount of antibodies, some experts are concerned that the presence of existing antibodies will inhibit the response to vaccination. However, according to the method of present example, it could be clearly confirmed that there was a vaccine reaction even after the administration of tixagevimab/cilgavimab (Figs. 4A and 4B).

The present inventors previously reported that in COVID-19 patients, mRNA expression of multiple BCR clonotypes was rapidly increased 2 weeks after onset and decreased within 1 week (Funakoshi Y, Ohji G, Yakushijin K, et al. Massive surge of mRNA expression of clonal B-cell receptor in patients with COVID-19. Heliyon. 2021; 7(8):e07748.). In this report, in order to measure the entire activity of humoral immunity using the BCR repertoire analysis method, it was considered to be important to perform analysis immediately after SARS-CoV-2 infection. However, according to the method of the present example, it was found that the frequency around 2 weeks after onset of symptoms was only 0.1 to 2% with respect to the SARS-CoV-2 specific sequence (Fig. 2B). That is, this result demonstrates that it is virtually impossible to determine a specific immune reaction to SARS-CoV-2 from the data of the BCR total repertoire by conventional repertoire analysis.

The majority of initial changes in total repertoire in infected individuals may be a result of inflammation associated with the infection or a secondary immune response. On the other hand, no significant changes based on total BCR repertoire data are observed in vaccinated volunteers (Fig. 7). However, according to the method of the present example, firstly, by focusing on the time of activation of humoral immunity in the primary or secondary immune reaction (several days to two weeks after antigen exposure), a plurality of blood samples were collected in a short period of time, and secondly, by performing "BCR repertoire data" analysis using "database of BCR sequences binding to target antigens", the reaction of vaccination by BCR repertoire was successfully demonstrated for each individual.

The characteristics of SARS-CoV-2 specific sequences were also investigated from the results according to the present example and from public databases. The SARS-CoV-2 specific sequences frequently included IGHV4-59 and short CDR3 amino acids. These sequences were also most frequently detected in multiple individuals and post-pandemic samples as a result of the present example, but some were also detected in samples prior to the 2019 pandemic, even at a much lower frequency. The sequence of IGHV4-59 forms a pair with the light chain of IGKV3-20/IGKJ1 and no neutralizing activity is reported. There is also a report that a public IGHV4-59/IGKV3 antibody binds to the S2 domain and cross-reacts with SARS-CoV-1. This sequence has the potential to react with a wide range of coronavirus strains. The known SARS-CoV-2 specific sequences are characterized by short CDR3 sequences and high pGen values. Since there is a large increase in sequences with high pGen values among known SARS-CoV-2 specific sequences after infection or vaccination, it is believed that the initial immune response upon exposure to unknown COVID-19 antigens is often made by a more abundant common SARS-CoV-2 specific sequence. According to the method of the present example, the known SARS-CoV-2 specific sequences of abundant high pGen values in all individuals can be utilized to reliably detect early infection immune responses.

Compared with the conventional method in which the vaccine reaction is evaluated by measuring the antibody titer by ELISA, the method of the present example has the following advantages.

First, since an antibody to be measured by the conventional method has a long half-life, the antibody remains in the blood for several months after being produced. On the other hand, since the activity of mRNA to be measured of the present invention is short-lived, it is possible to monitor the activity each time without being affected by SARS-CoV-2 vaccination, infection, antibody treatment, and the like.

In the conventional method, B cells are activated as humoral immunity, and there is a time lag until the antibody increases to a detectable level in the serum; however, in the method of the present example, the activity of B cells in the humoral immune reaction can be directly observed in real time. With these characteristics, the method of the present example can accurately evaluate humoral immunity, including primary and secondary responses. According to the present example, as a primary immune reaction, B cells were activated about 2 weeks after the first vaccination or initial exposure by infection. As a secondary immune reaction, B cells were activated about 1 week after booster vaccination.

While conventional serology cannot evaluate individual specific antibodies induced after infection, the method of the present example can provide useful information on individual antibodies. For example, according to the method of the present example, useful information on the characteristics of a plurality of antibodies having neutralizing/non-neutralizing activity can be obtained. The frequency of appearance of the neutralizing antibody sequence varies depending on the specimen, and is expected to be an index of immune protection of an infected individual. Since the appearance of an antibody having non-neutralizing activity is associated with the severity of infectious disease in patients with infectious diseases, detection of a non-neutralizing antibody is also an index to be noted. In recent years, as a method for evaluating the B cell response after infection or to a vaccine, an ELISpot assay for measuring human IgG-secreting B cells stimulated with R-848 and IL-2 in vitro has been developed, but such an ELISpot assay cannot provide a report of an immune response in real time or additional information.

It can be inferred that the usefulness of the method of the present example is not limited to confirmation of a vaccine reaction after administration of an antibody pharmaceutical, but is useful, for example, for estimating the degree of immune reconstitution after hematopoietic stem cell transplantation. Immune reconstitution after hematopoietic stem cell transplantation has been assessed by total lymphocyte count, lymphocyte subset analysis, cytokine profile analysis, but these assays are not accurate biomarkers. In general, almost all hematopoietic stem cell transplantation patients were vaccinated with mRNA SARS-CoV-2 vaccine at 3 to 6 months after transplantation. The method of the present example makes it possible to evaluate the reconstitution of humoral immunity at the timing of vaccination without being affected by prior infection, vaccination, or antibody treatment. The method of the present example can also be used to evaluate the reconstitution of humoral immunity following B cell depletion therapy.

As described above, it was demonstrated that antigen-specific antibody sequences can be quantified by BCR repertoire analysis using CoV-AbDab in order to evaluate the response to SARS-CoV-2 vaccination. According to the present example, it was found that there was also a reactivity to mRNA SARS-CoV-2 vaccination after the administration of tixagevimab/cilgavimab. The method of the present example can be applied to elucidation of a disease mechanism and development of a therapeutic agent in the field of antigen-antibody reaction.

### [Test Example 2]

The BCR/antibody sequences within CoV-AbDab are linked with information on strains (mutant strains and variants). For the BCR repertoire of Healthy volunteers 1 to 3, analysis was performed in the same manner as in Test Example 1 with respect to the sequence identical to the database (an antibody sequence having a Levenshtein distance in which the amino acid difference of CDR3 is 0 and an antibody sequence having a Levenshtein distance of 1 or less) immediately before vaccination with the mRNA vaccine against SARS-CoV-2 and on Day 7 and Day 14 after the vaccination, and a strain to which the antibody produced by the vaccine binds (mutant strain and variant of the omicron strain) was specified. The results are shown in Figs. 17A to 17C.

As shown in Figs. 17A to 17C, it was possible to specify a strain to which the antibody produced by the vaccine binds (mutant strain and variant of the omicron strain). When a new vaccine designed and developed targeting a new mutant strain or variant is similarly analyzed in place of the vaccine used in this test example, the new vaccine can also be used for functional evaluation as to whether or not the new vaccine binds to a known mutant strain or variant.

### [Test Example 3]

The same subject was vaccinated multiple times with an mRNA vaccine against SARS-CoV-2. A source strain vaccine was administered in the first and second times, a bivalent vaccine of the source strain and BA.4/5 was administered in the fifth time, and omicron XBB vaccine was administered in the sixth time. In the BCR repertoire immediately before the first vaccination, on Day 7 and Day 14 after the first vaccination, immediately before the second vaccination (Day 21 after the first vaccination), Day 3 and Day 7 after the second vaccination (Day 24 and Day 28 after the first vaccination), immediately before the fifth vaccination, Day 3 and Day 7 after the fifth vaccination, immediately before the sixth vaccination, and Day 7 and Day 12 after the sixth vaccination, the sequence identical to the database was analyzed in the same manner as in Test Example 1, and a strain to which the antibody produced by the vaccine binds (mutant strain and variant of the omicron strain) was specified. The results are shown in Fig. 18.

As shown in Fig. 18, an increase in identical sequences was observed after each vaccination. Analysis of each bindable mutant strain revealed that the number of antibody sequences bindable to the omicron strain was increased (in the figure, the increase indicated by the solid arrow in "Strains") and the number of antibody sequences bindable to XBB variants of the omicron strain was increased (in the figure, the increase indicated by the solid arrow in "Omicron sublineages") after vaccination with omicron XBB vaccine (6th). This confirmed the scientific validity (validity of target antigen for the type of strain (specifically, mutant strains/variants)) of the vaccinated omicron XBB vaccine.

As shown in Fig. 18, it was also found that the number of antibody sequences bindable to the omicron strain appearing thereafter was increased (in the figure, the increase indicated by the dotted arrow in "Strains") and the number of antibody sequences bindable to XBB variants of the omicron strain was increased (in the figure, the increase indicated by the dotted arrow in "Omicron sublineages") also by the vaccination with the source strain vaccine. As a result, it could be confirmed that the previously administered vaccine could also produce an antibody bindable to a strain (mutant strain or variant) that appears later. That is, it could be confirmed that the effectiveness of the past vaccines also applies to mutant strains that appear for the first time thereafter to some extent. It was also confirmed that effectiveness of the omicron XBB vaccine was higher for the omicron strain XBB variant, since there was a greater number of identical sequences for the omicron strain XBB variant in the case of vaccination with the omicron XBB vaccine than in the case of vaccination with the source strain vaccine.

### [Test Example 4]

Spike proteins that are targets of vaccines are composed of S1 and S2 parts, and it is known that the RBD region in S1 is important for infection. BNT-162b2 (Pfizer) and mRNA-1273 (Moderna) are mRNA vaccines targeting the entire spike proteins of S1 and S2 of SARS-CoV-2; on the other hand, MAFB-7256a (Daiichi Sankyo vaccine) approved in Japan in 2024 is an mRNA vaccine against RBD. The BCR/antibody sequences within the CoV-AbDab are linked with information on the epitope. For the BCR repertoire immediately before vaccination and on Days 7 and 14 after vaccination, sequences identical to the database were analyzed in the same manner as in Test Example 1, and epitopes were specified. The results are shown in Fig. 19. In Fig. 19, the solid arrow indicates a sequence in which the epitope is RBD (target of BNT-162b2 and MAFB-7256a), the dotted arrow indicates a sequence in which the epitope is S2 (target of BNT-162b2), and the broken arrow indicates a sequence in which the epitope is another site (a site where there is a possibility of RBD).

As shown in Fig. 19, the increased identical sequence on Day 7 after vaccination with BNT162b2 was for different regions of spike protein such as S1, RBD, or S2, while the increased sequence on Day 7 after vaccination with MAFB-7256a was for RBD. Therefore, the scientific validity (validity for the type of the target epitope) of the mRNA vaccine could be confirmed.

### [Test Example 5]

### [1] Time of clonal expansion of immune cells by infection, initial vaccination, or vaccine booster vaccination

For each of exposure to a specific antigen (SARS-CoV-2) by infection, exposure to a specific antigen (vaccine against SARS-CoV-2) by the initial vaccination with a vaccine, and exposure to a specific antigen (various vaccines against SARS-CoV-2) by the second or subsequent vaccination with a vaccine (booster vaccination), the sequence identical to the database in BCR repertoire (an antibody sequence having a Levenshtein distance in which the amino acid difference of CDR3 is 0 and an antibody sequence having a Levenshtein distance of 1 or less) was analyzed in time series in the same manner as in Test Example 1. The results are shown in Fig. 20A (primary reaction due to infection or primary vaccination) and Fig. 20B (secondary and subsequent reactions due to booster vaccination).

### [2] Time of clonal expansion of immune cells by cancer immunotherapy

Subset classification of B cells was performed, and it was confirmed that activated B cells (CD21 low B cells, class-switched B cells, plasma blasts, plasma cells) leading to antibody production increased at the same timing.

As shown in Fig. 21A (description of subset classification of B cells), activated B cells (CD21 low B cells, class-switched B cells, plasma blasts, plasma cells) leading to antibody production were analyzed. As shown in Figs. 21B and 21C, it was confirmed that the number of activated B cells increased 7 days after cancer immunotherapy (booster immunization). As shown in Fig. 21D, after the cancer immunotherapy (booster immunization), activated B cells increased at 7 days, and a larger increase in activated B cells was further observed in cases (specifically, Patients 1, 2, 6, 10, and 11) who developed irAE (side effect: autoimmune disease after administration of an immune checkpoint inhibitor).

### [3] Results

As shown in Fig. 20A, after infection or initial vaccination, the sequence identical to the database in BCR repertoire increased in about 2 weeks, and as shown in Fig. 20B, after booster vaccination, the sequence identical to the database in BCR repertoire increased in about 1 week. As shown in Figs. 21B and 21D, activated B cells increased in about 1 week even after cancer immunotherapy (booster immunization). That is, it was confirmed that the time of clonal expansion of immune cells was commonly about 2 weeks after antigen stimulation in the case of a primary reaction of an immune reaction, and about 1 week after antigen stimulation in the case of a secondary reaction of an immune reaction.

### [Test Example 6]

The immune reaction by vaccination to Patients 1 to 3 after hematopoietic stem cell transplantation was evaluated. Figs. 22A and 22B show the background and analysis results of Patients 1 to 3.

Patient 1 was subjected to hematopoietic stem cell transplantation after a single infection with SARS-CoV-2, and then vaccinated with a vaccine (specific antigen) against SARS-CoV-2. Patient 2 was subjected to hematopoietic stem cell transplantation after two infections with SARS-CoV-2, and then vaccinated with a vaccine (specific antigen) against SARS-CoV-2. Patient 3 was subjected to hematopoietic stem cell transplantation after two infections with SARS-CoV-2, and then vaccinated with a vaccine (specific antigen) against SARS-CoV-2 after three infections with SARS-CoV-2. In the BCR repertoire obtained from the specimen collected at the time point of "*" in Figs. 22A and 22B, the sequences identical to the database (an antibody sequence having a Levenshtein distance in which the amino acid difference of CDR3 is 0 and an antibody sequence having a Levenshtein distance of 1 or less) were analyzed in the same manner as in Test Example 1.

As shown in Fig. 22A, regardless of the antigen stimulation before the hematopoietic stem cell transplantation and the number of times thereof, the identical sequences were increased by a first antigen stimulation (vaccination) after hematopoietic stem cell transplantation in 2 weeks after the first antigen stimulation. As shown in Fig. 22 B, regardless of the antigen stimulation before the hematopoietic stem cell transplantation, the identical sequences were increased by a second or subsequent antigen stimulation (vaccination) after the hematopoietic stem cell transplantation in 1 week from the second or subsequent antigen stimulation. That is, it was also confirmed that the immunity acquired by the antigen stimulation before the hematopoietic stem cell transplantation was reset by the hematopoietic stem cell transplantation. Therefore, it is also possible to determine whether the immune reaction to be analyzed is caused by either the first antigen stimulation or the second or subsequent antigen stimulation after the hematopoietic stem cell transplantation, depending on the timing of clonal expansion of immune cells (increase in identical sequences) by antigen stimulation after the hematopoietic stem cell transplantation. That is, when the timing is about 2 weeks after the antigen stimulation after the hematopoietic stem cell transplantation, the immune reaction to be analyzed can be determined to be due to the first antigen stimulation after the hematopoietic stem cell transplantation, and when the timing is about 1 week after the antigen stimulation after the hematopoietic stem cell transplantation, the immune reaction to be analyzed can be determined to be due to the second or subsequent antigen stimulation after the hematopoietic stem cell transplantation.

### [Test Example 7]

About 1300 sequences of the antigen recognition site of the B cell receptor bindable to the influenza HA antigen were independently collected and a database was created by citation from the article. As a result of analyzing the immune reaction (the immune reaction by the vaccination is the secondary and subsequent reactions since the subject is already infected with influenza) BCR repertoire after the influenza inactivated vaccination using this database, as shown in Fig. 23A, an increase in identical sequences 1 week after the vaccination (immune reaction by stimulation of the influenza HA antigen) could be confirmed. The sequences were collected by in vitro experiment shown in Fig. 23B and this database was further expanded. The database further expanded by continuing sequence collection can be similarly used for evaluating the immune reaction.

## Claims

1. A method for evaluating an immune reaction, the method comprising:
step A of preparing repertoire data including a group of sequences of antigen recognition sites in immune cell receptors, the repertoire data being acquired from a specimen of a subject; and
step B of collating database including a group of sequences of antigen recognition sites in immune cell receptors for a specific antigen with the repertoire data to detect, from the repertoire data, a sequence which is identical to the sequence included in the database at a level of amino acid sequences or a sequence which is identical to the sequence included in the database at a portion excluding two or less amino acid residues.

2. The method according to claim 1, further comprising step C of deriving the number and/or frequency of sequences detected in the step B in the repertoire data.

3. The method according to claim 2, further comprising step D of confirming a temporal change in the number and/or frequency.

4. The method according to claim 3, wherein it is confirmed in the step D whether a time when an increase is observed as the temporal change corresponds to 11 to 20 days or 3 to 10 days after stimulation with the specific antigen.

5. The method according to any one of claims 1 to 4, wherein the subject is a subject exposed to the specific antigen.

6. The method according to claim 5, wherein the exposure to the specific antigen is vaccination with a vaccine.

7. The method according to claim 6, wherein the vaccine is a nucleic acid vaccine.

8. The method according to claim 6, wherein the vaccine is a coronavirus vaccine or an influenza vaccine.

9. The method according to claim 7, further comprising step E of evaluating the scientific validity of the nucleic acid vaccine by confirming additional information listed in the database for the sequence in which an increase is confirmed as the temporal change in the number and/or frequency, wherein
the additional information is selected from the group consisting of [1] information on the presence or absence of neutralizing activity against an antigen, [2] information on a species or strain of an antigen, and [3] information on an epitope, and
the evaluating of the scientific validity is selected from the group consisting of [1] evaluating that the nucleic acid vaccine has clinical effectiveness of producing an antibody having neutralizing activity when it is confirmed that the neutralizing activity exists, [2] evaluating that the nucleic acid vaccine is compatible with a target antigen when it is confirmed that the species or strain is the same as a species or strain of the target antigen in design of the nucleic acid vaccine, and [3] evaluating that the nucleic acid vaccine is compatible with a target epitope when it is confirmed that the epitope is the same as the target epitope in design of the nucleic acid vaccine.

10. The method according to claim 7, further comprising step E' of evaluating that the nucleic acid vaccine has an ability to produce an antibody having an antigen recognition site completely identical to that of an antibody by adaptive immunity by infection, as the scientific validity of the nucleic acid vaccine, when the sequence in which an increase is confirmed as the temporal change in the number and/or frequency is identical to the sequence included in the database at a level of amino acid sequences.

11. The method according to claim 5, wherein the exposure to the specific antigen is infection.

12. The method according to claim 11, wherein the specific antigen is a coronavirus or an influenza virus.

13. The method according to claim 5, wherein the subject is a subject administered with an antibody drug, vaccinated with a vaccine, and/or infected, and
the antibody drug, the vaccine, and a pathogen of the infection allow another antibody different from an antibody produced by exposure to the specific antigen to be retained in a body of the subject.

14. The method according to claim 13, wherein the specific antigen is a vaccine, and
the method further comprises step F of determining that the vaccine works when the number and/or frequency increases over time.

15. The method according to claim 5, wherein the specimen is collected at a time of activation of mRNA of an immune cell receptor for the specific antigen.

16. The method according to claim 15, wherein the time of activation is 11 to 20 days or 3 to 10 days after the exposure.

17. The method according to any one of claims 1 to 3, wherein the subject is a subject subjected to cancer immunotherapy.

18. The method according to claim 16, wherein the specimen is collected at a time of activation of mRNA of an immune cell receptor for the specific antigen.

19. The method according to claim 17, wherein the time of activation is 11 to 20 days or 3 to 10 days after the cancer immunotherapy.

20. The method according to any one of claims 1 to 4, wherein the subject is a subject subjected to an immunosuppressive treatment.

21. The method according to claim 20, wherein the immunosuppressive treatment is hematopoietic stem cell transplantation or administration of a B cell depletion therapy.

22. The method according to claim 21, wherein the immunosuppressive treatment is hematopoietic stem cell transplantation or a B cell depletion therapy, and the method further comprises step G of determining the presence or absence of restoration of immune function on the basis of whether or not the temporal change in the number and/or frequency has increased.

23. The method according to any one of claims 1 to 4, wherein the subject is a patient with an autoimmune disease.

24. The method according to claim 1, wherein the repertoire data is acquired from a specimen of a subject vaccinated with a vaccine against a previously-encountered viral strain ST1,
the specific antigen is a viral strain ST2 for which effectiveness by the vaccine is unknown, and
the method further comprises step H of evaluating that the vaccine is also effective against the viral strain ST2 when the detected sequence is present.

25. The method according to claim 1, wherein the database includes sequences of antigen recognition sites in the immune cell receptors for the specific antigen, the sequences of antigen recognition sites in the immune cell receptors collected from a population consisting of objects to be examined in which an immune reaction by the specific antigen has occurred, and the database is obtained by the following steps:
a step of acquiring, from each specimen of the objects to be examined, repertoire data in time series at a time Tₑₓ of activation of mRNA of an immune cell receptor for the specific antigen, a time T_{bf} before the time of activation and a time T_{af} after the time of activation, the repertoire data including a group of sequences of antigen recognition sites in the immune cell receptors; and
a step of selecting a sequence of an antigen recognition site in an immune cell receptor at which proliferation is observed at the time Tₑₓ of activation as information to be collected in the database.

26. The method according to claim 25, wherein the time Tₑₓ of activation is 11 to 20 days or 3 to 10 days after stimulation with the specific antigen.

27. The method according to claim 1, wherein the immune cell is a T cell or a B cell.
